# EUROPEAN PATENT SPECIFICATION

(11) **EP 0 977 755 B1**
(45) Date of publication and mention of the grant of the patent: **13.07.2005**
(21) Application number: 98914848.1
(22) Date of filing: 15.04.1998
(51) Int. Cl.: C07D 471/08, C07D 453/02, C07D 487/08, A61K 31/41

(54) **HETEROCYCLIC COMPOUNDS AND THEIR PREPARATION AND USE**
HETEROZYKLISCHE VERBINDUNGEN, IHRE HERSTELLUNG UND IHRE ANWENDUNG
COMPOSES HETEROCYCLIQUES, LEUR PREPARATION ET LEUR UTILISATION

(30) Priority: 22.04.1997 DK 45597; 29.09.1997 DK 111497
(43) Date of publication of application: 09.02.2000
(73) Proprietor: NOVO NORDISK A/S, 2880 Bagsvaerd (DK)
(72) Inventor: JEPPESEN, Lone, DK-2830 Virum (DK); SAUERBERG, Per, DK-3520 Farum (DK)
(86) International application number: PCT/DK1998/000156
(87) International publication number: WO 1998/047900

(56) References cited:
- WO-A-94/20496
- WO-A-97/40045
- US-A- 5 527 813

## Description

### Field of the Invention

The present invention relates to therapeutically active azabicyclic compounds, a method of preparing the same and to pharmaceutical or veterinary compositions comprising the compounds The novel compounds are useful in treating a disease in the central nervous system caused by malfunctioning of the muscarinic cholinergic system

### Background of the Invention

Due to the generally improved health situation in the western world, elderly-related diseases are much more common now than in the past and are likely to be even more common in the future.

One of the elderly-related symptoms is a reduction of the cognitive functions. This symptom is especially pronounced in the pathophysiological disease known as Alzheimer's disease. This disease is combined with, and also most likely caused by, an up to 90% degeneration of the cholinergic neurons in nucleus basalis, which is part of substantia innominata. These neurons project to the prefrontal cortex and hippocampus and have a general stimulatory effect on the cognitive functions of the forebrain as well as of hippocampus, namely learning, association, consolidation, and recognition.

It is a charactenstic of Alzheimer's disease that although the cholinergic neurons degenerate, the postsynaptic receptors in the forebrain and hippocampus still exist. Therefore cholinergic agonists are useful in the treatment of Alzheimer's disease, in halting progression of Alzheimer's disease, and in improving the cognitive functions of elderly people.

Compounds active at a muscarinic cholinergic receptor are also useful analgesic agents and therefore are useful in the treatment of severely painful conditions.

Furthermore, the compounds of this invention are useful in the treatment of glaucoma, psychosis, mania, bipolar disorder, schizophrenia or schizophreniform conditions, depression, bladder dysfunctions, anxiety, sleeping disorders, epilepsy, cerebral ischemia and gastrointestinal motility disorders.

WO 92/03433 and WO 94/20496 disclose therapeutically active azabicycfic compounds which are substituted by either a 1,2,5-thiadiazole or a 1,2,5-oxadiazole ring system. The compounds disclosed therein are active at the muscarinic cholinergic receptors

Within the muscarinic cholinergic pharmacology five subtypes of muscarinic cholinergic receptors exist which are M₁ through M₅
Some muscarinic cholinergic receptor active compounds are associated with side effects attributed to undesired modulation of the muscarinic cholinergic receptors

Therefore, new compounds having muscarinic cholinergic activity are desired which have a better combination of receptor subtype efficacy, potency and selectivity

The presently claimed compounds have a surprisingly good combination of M, receptor efficacy, potency and selectivity

### Summary of the Invention

It is an object of the invention to provide new muscannic cholinergic compounds.

The novel compounds of the invention are heterocyclic compounds having the formula I 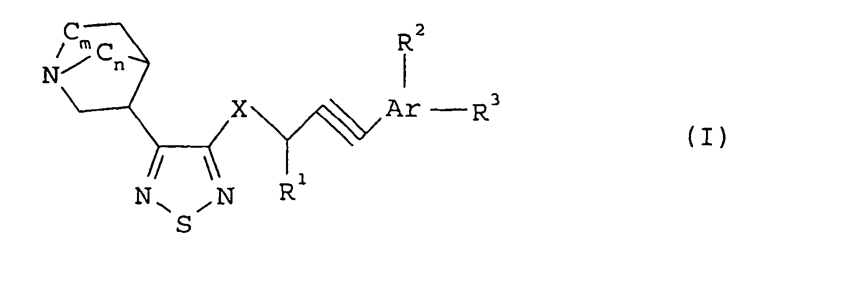 wherein
X is oxygen or sulphur; and
R¹ is hydrogen, straight or branched C₁₋₅-alkyl, straight or branched C₂₋₅-alkenyl, straight or branched C₂₋₅-alkynyl or straight or branched C₄₋₅-alkenynyl, each of which is optionally substituted with one or more halogen(s); and
R² and R³ are independently hydrogen, halogen, CN, NO₂, CF₃, OCF₃, C₁₋₃-alkyl, C₁₋₃-alkoxy or C₁₋₃-alkylthio, wherein C₁₋₃-alkyl, C₁₋₃-alkoxy and C₁₋₃-alkylthio are optionally substituted with one or more halogen(s), cyano, amino or nitro, and
Ar is phenyl, thienyl, pyridyl, pyrimidinyl, thiazolyl or furyl; and
n is 0, 1 or 2; and
m is 0, 1 or 2; or
a pharmaceutically acceptable salt or solvate thereof.

As used herein, the term "halogen" means F, Cl, Br and I. Especially preferred halogens include Cl, Br and F.

The term "C_{1-n'}-alkyl" wherein n' can be from 2 through 5, as used herein, represent a branched or straight alkyl group having from one to the specified number of carbon atoms. Typical C₁₋₄-alkyl groups include, but are not limited to, methyl, ethyl, n-propyl, iso-propyl, butyl, iso-butyl, sec-butyl, tert-butyl and the like.

The term "C_{2-n'}-alkenyl" wherein n' can be from 3 through 5, as used herein, represents an olefinically unsaturated branched or straight group having from 2 to the specified number of carbon atoms and at least one double bond. Examples of such groups include, but are not limited to, 1-propenyl, 2-propenyl, 1,3-butadienyl, 1-butenyl, pentenyl, and the like.

The term "C_{2-n'}-alkynyl" wherein n' can be from 3 through 5, as used herein, represent an unsaturated branched or straight group having from 2 to the specified number of carbon atoms and at least one triple bond. Examples of such groups include, but are not limited to, 1-propynyl, 2-propynyl, 1-butynyl, 2-butynyl, 1-pentynyl, 2-pentynyl and the like.

The term "C₄₋₅-alkenynyl" as used herein, represent an unsaturated branched or straight hydrocarbon group having from 4 to 5 carbon atoms and both at least one double bond and at least one triple bond. Examples of such groups include, but are not limited to, 1-penten-4-yne, 3-penten-1-yne and the like.

The term "C₁₋₃-alkoxy" as used herein, alone or In combination, refers to a straight or branched monovalent substituent comprising a C₁₋₃-alkyl group linked through an ether oxygen having its free valence bond from the ether oxygen and having 1 to 3 carbon atoms. Examples of such groups include, but are not limited to, e g. methoxy, ethoxy, propoxy, isopropoxy and the like.

The term "C₁₋₃-alkylthio" as used herein, alone or in combination, refers to a straight or branched monovalent substituent compnsing a C₁₋₃-alkyl group linked through a divalent sulfur atom having its free valence bond from the sulfur oxygen and having 1 to 3 carbon atoms. Examples of such groups include, but are not limited to, e g. methylthio, ethylthio, propylthio, isopropylthio, butylthio and the like.

As used herein, the phrase "one or more selected from" shall more preferably refer to from 1-3 substituents. The term shall further preferably refer to from 1-2 substituents.

In a preferred embodiment, the present invention is concerned with compounds of formula I wherein X is oxygen.

In another preferred embodiment, the present invention is concerned with compounds of formula I wherein X is sulfur.

In another preferred embodiment, the present invention is concerned with compounds of formula I wherein R¹ is hydrogen.

In a further preferred embodiment, the present invention is concerned with compounds of formula I wherein R¹ is straight or branched C₁₋₅-alkyl, preferably straight or branched C₁₋₃-alkyl.

In a further preferred embodiment, the present invention is concerned with compounds of formula I wherein R¹ is isopropyl.

In a further preferred embodiment, the present invention is concerned with compounds of formula I wherein R² and R³ are halogen

In a further preferred embodiment, the present invention is concerned with compounds of formula I wherein Ar is phenyl or thienyl.

In a further preferred embodiment, the present invention is concerned with compounds of formula I wherein m is 1 or 2.

In a further preferred embodiment, the present invention is concerned with compounds of formula I wherein n is 1 or 2.

It is to be understood that the invention extends to each of any of the stereoisomeric forms of the compounds of the present invention as well as the pure diastereomeric, pure enantiomeric, and racemic forms of the compounds of this invention.

The starting materials for the illustrated process are, if nothing else mentioned, commercially available or may be prepared using methods known to the skilled artisan The invention also relates to methods of preparing the above mentioned compounds, comprising:
a) The propargyl side chains can be prepared by standard cross coupling methods using iodine or bromine substituted aromates and propargylalcohols in the presence of a palladium catalyst e g.: 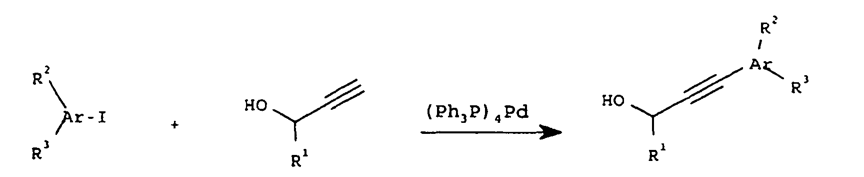 wherein Ar, R¹, R² and R³ have the meanings defined above which alcohol can be reacted with an azabicydic 1,2,5-thiadiazole under basic conditions 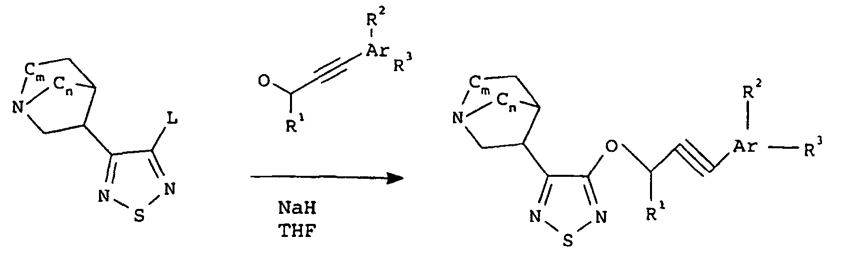 wherein L is a leaving group e g chlorine or SO₂R, wherein R is alkyl or phenyl and Ar, R¹, R², R³, n and m have the meanings defined above; or
b) compounds wherein X is S can be synthesized by displacing the leaving group L with sulfur e g. using NaSH, and then alkylating on the sulfur atom with P-propargylalcohol, wherein O-P is a leaving group e g O-SO₂R or O-"Mitsunobu", wherein R has the meaning defined above, 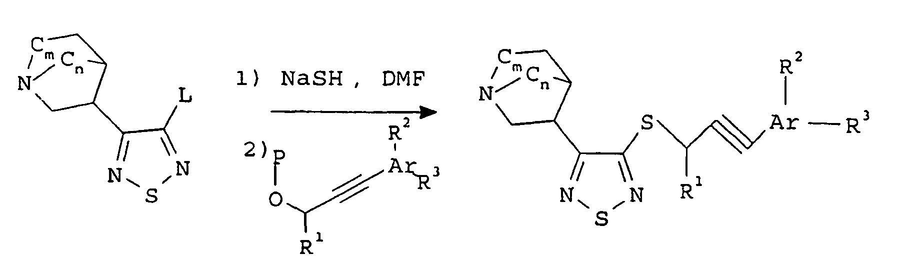 and wherein Ar, R¹, R², R³, n and m have the meanings defined above.

As is always the case in chemistry, the rate of the reaction depends on a variety of factors, such as the temperature and the exact compound which is to be prepared. The course of the reaction may be followed using methods such as thin layer chromatography (TLC), high performance liquid chromatography (HPLC), gas chromatography (GC) and nuclear magnetic resonance spectroscopy (NMR) to detect the degree of completion of the reaction. The operator may obtain maximum yields using the process by extending the reaction time. Alternatively, the operator may wish to obtain maximum throughput by cutting off the reaction at the point at which it reaches an economical degree of completion.

When the product of a step in the following process is an oil, it may be isolated by standard methods. Such methods include distillation, flash chromatography, HPLC and the like.

The invention further provides a formulation comprising a compound of formula I and one or more pharmaceutically acceptable diluents, carriers or excipients therefor.

The invention provides the use of a compound of formula (I) or a pharmaceutically acceptable salf thereof for the preparation of a medicament for treating a condition associated with a malfunction of the cholinergic muscarinic receptor system. Such conditions which may be treated using a compound of this invention include, but are not limited to Alzheimer's Disease, cognitive dysfunction, severely painful conditions, glaucoma, psychosis, schizophrenia, bladder dysfunction, anxiety, sleep disorders, and other such conditions associated with the modulation of a muscarmc receptor.

As used herem the term "treating" Includes prophylaxis of a physical and/or mental condition or amelioration or elimination of the developed physical and/or mental condition once it has been established or alleviation of the characteristic symptoms of such condition

As used herein the term "malfunctioning of the muscarinic cholinergic system" shall have the meaning accepted by the skilled artisan. For example the term shall refer to, but is not in any way limited to conditions such as glaucoma, psychosis, schizophrema or schizophreniform conditions, depression, sleeping disorders, epilepsy and gastrointesbnal motility disorders. Other such conditions include Alzheimer's disease and incontinence.

As used herein the phrase "interacting with a muscarinic cholinergic receptor" shall include compounds which block muscannic cholinergic receptors or modulate such receptors. The phrase shall Include the effect observed when compounds act as agonists, partial agonists and/or antagonists at a muscarinic cholinergic receptor.

Examples of pharmaceutically acceptable salts include inorganic and organic acid addition salts such as hydrochlonde, hydrobromide, sulphate, phosphate, acetate, fumarate, maleate, citrate, lactate, tartrate, oxalate, or similar pharmaceutically acceptable inorganic or organic acid addition salts, and include the pharmaceutically acceptable salts listed in Journal of Pharmaceutical Science, **66,** 2 (1977) which are known to the skilled artisan. The compounds of this invention may form solvates with standard low molecular weight solvents using methods known to the skilled artisan.

The pharmacological properties of the compounds of the invention can be illustrated by determining the M, efficacy and potency of the compounds of the present invention in A9 L-m, cells.

IP hydrolysis in A9 L cells cloned with the genetic m, muscarinic receptor:
A9 L cells transfected with human and rat m, muscarinic receptors (Dörje et al., 1991; Jones et al., 1988), were cultured to confluence in 75-ml flasks containing Dulbecco's modified essential media. Cells were prelabeled with 1 µCi/ml of myo(2-[³H]inositol (Amersham Inc., 16.3 Ci/mmol) for 48 hr before assaying. On the day of assay, cells were detached using a 30-sec exposure to 0.25% trypsin in 1 mM EDTA. The cells were collected by centrifugation (300 x g for 5 min) and resuspended in oxygenated HEPES buffer containing 10 mM LiCl (NaCl 142 mM, KCl 5.6 mM, CaCl₂ 2.2 mM, MgCl₂ 1 mM, NaHCO₃ 3.6 mM, D-glucose 5.6 mM and Na⁺ HEPES 30 mM, pH 7.4). Cells were incubated at 37°C for 45 min in the presence of varying concentrations of test compounds. Total IP hydrolysis was determined using the method of Schoepp and Johnson (1988). Data are expressed as the percentage of total [³H]IP accumulated in the presence of 100 µM carbachol stimulation. Half-maximal (EC₅₀) values were determined from the mean (±S.D.) of seven point curves determined from three separate experiments.

Test results obtained by testing some compounds of the present invention will appear from the following table 1.

**Table 1**

| | | A9 L- m₁ cells |
|---|---|---|
| Compound no | % PI (efficacy) | EC₅₀, nM |
| 1 | 80 | 210 |
| 2 | 74 | 1800 |
| 3 | 81 | 95 |
| 4 | 77 | 159 |
| 5 | 73 | 42 |
| 7 | 108 | 380 |
| 8 | 91 | 670 |
| 9 | 68 | 38 |
| 10 | 57 | 75 |
| 11 | 64 | 98 |
| 13 | 100 | 0.7 |
| 16 | 56 | 14 |
| 20 | 98 | 1 |
| 21 | 67 | 105 |
| 24 | 105 | 300 |
| 25 | 69 | 210 |
| 26 | 93 | 280 |
| 27 | 79 | 187 |
| 28 | 97 | 22 |
| 30 | 82 | 20 |
| 31 | 108 | 26 |
| 39 | 105 | 0.2 |
| 40 | 102 | 0.5 |
| 41 | 100 | 2 |
| 42 | 99 | 12 |
| 43 | 85 | 9 |
| 44 | 81 | 32 |
| 45 | 66 | 8 |
| 47 | 102 | 102 |
| 48 | 96 | 7 |
| 49 | 100 | 0.7 |
| 50 | 101 | 2 |
| 53 | 97 | 13 |
| 54 | 102 | 224 |
| Carbachol | 99 | 4600 |
| oxotremorine | 88 | 16300 |

The compounds of the invention are effective over a wide dosage range. For example, in the treatment of adult humans, dosages from about 0 05 to about 100 mg, preferably from about 0 1 to about 100 mg, per day may be used. A most preferable dosage is about 0.1 mg to about 70 mg per day. In choosing a regimen for patients suffering from diseases in the central nervous system caused by malfunctioning of the muscarinic cholinergic system it may frequently be necessary to begin with a dosage of from about 20 to about 70 mg per day and when the condition is under control to reduce the dosage as low as from about 0 1 to about 10 mg per day. The exact dosage will depend upon the mode of administration, form in which administered, the subject to be treated and the body weight of the subject to be treated, and the preference and experience of the physician or veterinarian in charge

The route of administration may be any route, which effectively transports the active compound to the appropriate or desired site of action, such as oral or parenteral e.g. rectal, transdermal, depot, subcutaneous, intravenous, intraurethral, intramuscular, intranasal, ophthalmic solution or an ointment, the oral route being preferred.

Typical compositions include a compound of formula I or a pharmaceutically acceptable acid addition salt thereof, associated with a pharmaceutically acceptable excipient which may be a carrier or a diluent or be diluted by a carner, or enclosed within a carner which can be in the form of a capsule, sachet, paper or other container. In making the compositions, conventional techniques for the preparation of pharmaceutical compositions may be used. For example, the active compound will usually be mixed with a carrier, or diluted by a carrier, or enclosed within a carrier which may be in the form of a ampoule, capsule, sachet, paper, or other container. When the carrier serves as a diluent, it may be solid, semi-solid, or liquid material which acts as a vehicle, excipient, or medium for the active compound. The active compound can be adsorbed on a granular solid container for example in a sachet. Some examples of suitable carriers are water, salt solutions, alcohols, polyethylene glycols, polyhydroxyethoxylated castor oil, gelatine, lactose, amylose, magnesium stearate, talc, silicic acid, fatty acid monoglycendes and diglycerides, pentaerythritol fatty acid esters, hydroxymethylcellulose and polyvinylpyrrolidone. The formulations may also include wetting agents, emulsifying and suspending agents, preserving agents, sweetening agents or flavouring agents The formulations of the invention may be formulated so as to provide quick, sustained, or delayed release of the active ingredient after administration to the patient by employing procedures well known in the art.

The pharmaceutical preparations can be stenlized and mixed, if desired, with auxiliary agents, emulsifiers, salt for influencing osmotic pressure, buffers and/or colouring substances and the like, which do not deleteriously react with the active compounds.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, corn starch, and/or potato starch. A syrup or elixir can be used in cases where a sweetened vehicle can be employed.

Generally, the compounds are dispensed in unit form comprising from about 0.1 to about 100 mg in a pharmaceutically acceptable carrier per unit dosage.

A typical tablet, appropriate for use in this method, may be prepared by conventional tabletting techniques and contains:

| | |
|---|---|
| Active compound | 5.0 mg |
| Lactosum | 67.8 mg Ph.Eur. |
| Avicel® | 31.4 mg |
| Amberlite® | 1.0 mg |
| Magnesii stearas | 0.25 mg Ph. Eur. |

The compounds according to this invention may be suitable for administration to an animal. Such animals include both domestic animals, for example livestock, laboratory animals, and household pets, and non-domestic animals such as wildlife. More preferably, the animal is a vertebrate. Most preferably, a compound according to this invention shall be administered to a mammal. It is especially preferred that the animal is a domestic mammal or a human. The most preferred mammal is a human. For such purposes, a compound of this invention may be administered as a feed additive or in bulk form.

The invention will now be described in further detail with reference to the following examples. The examples are provided for illustrative purposes, and are not to be construed as limiting the scope of the invention in any way.

The absolute configuration is not known for all of the following examples The use of α in the nomenclature refers to the starting material having (+) configuration, whereas the use of β refers to the starting material having (-) configuration.

### Example 1

### Endo (+-) 3-(3-(3-Phenyl-2-propyn-1-yloxy)-1,2,5-thiadiazol-4-yl)-1-azabicyclo[2 2.1]heptane,(+)--tartrate.

To a mixture of sodium hydride (60% in mineral oil, 0.63 g, 15 mmol) in THF (50ml) was added 3-phenyl-2-propyn-1-ol (0.62 g, 0.47 mmol) at room temperature and the reaction mixture was stirred for 1 hour. After cooling to 0 °C, endo (+-) 3-(3-propylsulfonyl-1,2,5-thiadiazol-4-yl)-1-azabicyclo[2.2.1]heptane (WO 94/20496) (0.48 g, 1.66 mmol) was added. The reaction mixture was stirred for 5 hours at room temperature and at 5 °C overnight. Water (50 ml) was added and the mixture was extracted with ether (3x 100 ml). The ether phases were evaporated and the residue was dissolved in 1 N hydrochloric acid (20 ml). The water phase was washed with ether (100 ml), made basic with 25% aqueous ammonia and then extracted with ether (2x100 ml). The ether phases were dried and evaporated to give crude product. Purification on column chromatography eluting with ethyl acetate:methano1:25%aq.NH₃ (2:1:2%) gave the desired free base product as an oil. Crystallization with L-(+)-tartaric acid from isopropanol gave the title compound in 300mg (39%) yield. Mp. 122-125°C. Compound 1.

The following compound was made in the same manner using the appropriate alcohol:
Endo (+-) 3-(3-(3-(4-Fluorophenyl)-2-propyn-1-yloxy)-1,2,5-thiadiazol-4-yl)-1-azabicyclo[2.2.1]heptane,(+)-tartrate. Mp. 130-132 °C. Compound 2.

### Example 2

### Endo (α)-3-(3-(3-(4-Fluorophenyl)-2-propyn-1-yloxy)-1.2.5-thiadiazol-4-yl)-1-azabicyclo[2.2.1]heptane.(+)-tartrate.

To a mixture of sodium hydride (60% in mineral oil, 0.5 g, 12.5 mmol) in THF (50ml) was added 3-(4-fluorophenyl)-2-propyn-1-ol (0.42 g, 2.8 mmol) at room temperature and the reaction mixture was stirred for 1 hour. After cooling to 0 °C, endo (+) 3-(3-propylsulfonyl-1,2,5-thiadiazol-4-yl)-1-azabicyclo[2.2.1]heptane (WO 94/20496) (0.47 g, 1 66 mmol) was added. The reaction mixture was stirred for 5 hours at room temperature and at 5 °C overnight. Water (50 ml) was added and the mixture was extracted with ether (3x 100 ml). The ether phases were evaporated and the residue was dissolved in 1 N hydrochloric acid (20 ml). The water phase was washed with ether (100 ml), made basic with 25% aqueous ammonia and then extracted with ether (2x100 ml). The ether phases were dried and evaporated to give crude product (0.41 g). Purification on column chromatography eluting with ethyl acetate:methanol:25%aq.NH₃ (2:1:2%) gave the desired free base product as an oil.

Crystallization with L-(+)-tartaric acid (204 mg, 1.36 mmol) from isopropanol gave the title compound in 575mg (75%) yield. Mp. 141-142°C. Compound 3.

The following compounds were made in exactly the same manner using the appropriate alcohol:
Endo (α)-3-(3-(3-Phenyl-2-propyn-1-yloxy)-1,2,5-thiadiazol-4-yl)-1-azabicyclo[2.2.1]heptane, (+)-tartrate. Mp. 127-130°C. Compound 4.
Endo (α)-3-(3-(3-(3-Methoxyphenyl)-2-propyn-1-yloxy)-1,2,5-thiadiazol-4-yl)-1-azabicyclo[2.2.1]heptane, (+)-tartrate. Mp 139-141°C. Compound 5.
Endo (α)-3-(3-(3-(4-Chlorophenyl)-2-propyn-1-yloxy)-1,2,5-thiadiazol-4-yl)-1-azabicyclo[2.2 1]heptane, (+)-tartrate. Mp. 163-164°C. Compound 6.
Endo (α)-3-(3-[3-(3-Thienyl)-2-propyn-1-yloxy]-1,2,5-thiadiazol-4-yl)-1-azabicyclo[2.2 1]heptane, (+)-tartrate. Mp. 141.5-143°C. Compound 7.
Endo (α)-3-(3-(3-(2-Thienyl)-2-propyn-1-yloxy)-1,2,5-thiadiazol-4-yl)-1-azabicyclo[2 2 1]heptane. (+)-tartrate. Mp. 113-115°C. Compound 8
Endo (α)-3-(3-(3-(3,5-Difluorophenyl)-2-propyn-1-yloxy)-1,2,5-thiadiazol-4-yl)-1-azabicyclo[2.2.1]heptane, (+)-tartrate. Mp. 158-160°C. Compound 56.
Endo (α)-3-(3-(3-(3-Chloro-5-fluorophenyl)-2-propyn-1-yloxy)-1,2,5-thiadiazol-4-yl)-1-azabicyclo[2.2.1]heptane, (+)-tartrate. Mp. 120-121°C. Compound 57.

### Example 3

### Endo (β)-3-(3-(3-(4-Fluorophenyl)-2-propyn-1-yloxy)-1.2.5-thiadiazol-4-yl)-1-azabicyclo[2 2.1]heptane, (-)-tartrate.

To a mixture of sodium hydnde (60% in mineral oil, 0.63 g, 15 mmol) in THF (50ml) was added 3-(4-fluorophenyl)-2-propyn-1-ol (0.70 g, 4.7 mmol) at room temperature and the reaction mixture was stirred for 1 hour. After cooling to 0 °C, endo (-) 3-(3-propylsulfonyl-1,2,5-thiadiazol-4-yl)-1-azabicyclo[2.2.1]heptane (WO 94/20496) (0 48 g, 1.66 mmol) was added. The reaction mixture was stirred for 5 hours at room temperature and at 5 °C overnight. Water (50 ml) was added and the mixture was extracted with ether (3x 100 ml). The ether phases were evaporated and the residue was dissolved in 1 N hydrochloric acid (20 ml). The water phase was washed with ether (100 ml), made basic with 25% aqueous ammonia and then extracted with ether (2x100 ml). The ether phases were dried and evaporated to give crude product (480 mg). Crystallisation with D-(-) tartaric acid (240 mg, 1.6 mmol) from isopropanol gave the title compound in 450mg (57%) yield. Mp. 85-88°C. Compound 9.

The following compounds were made in the same manner using the appropnate alcohol and appropnate acid
Endo (β)-3-(3-(3-(3-Methoxyphenyl)-2-propyn-1-yloxy)-1,2,5-thiadiazol-4-yl)-1-azabicyclo[2.2.1]heptane, (-)-tartrate. Mp. 124-127°C. Compound 10.
Endo (β)-3-(3-(3-Phenyl-2-propyn-1-yloxy)-1,2,5-thiadiazol-4-yl)-1-azabicyclo[2.2.1]heptane, (+)-tartrate. Mp. 150-153°C . Compound 11.
Endo (β)-3-(3-(3-(3-Furyl)-2-propyn-1-yloxy)-1,2,5-thiadiazol-4-yl)-1-azabicyclo[2.2.1]heptane, (-)-tartrate. Mp. 129-131°C. Compound 58.
Endo (β)-3-(3-(3-(3-Cyanophenyl)-2-propyn-1-yloxy)-1,2,5-thiadiazol-4-yl)-1-azabicyclo[2.2.1]heptane, (-)-tartrate. Mp. 118-120°C. Compound 59.
Endo (β)-3-(3-(3-(3-Trifluoromethoxyphenyl)-2-propyn-1-yloxy)-1,2,5-thiadiazol-4-yl)-1-azabicyclo[2.2 1]heptane, (+)-tartrate. Mp. 54-55°C Compound 60.
Endo (β)-3-(3-(3-(3-Chlorophenyl)-2-propyn-1-yloxy)-1,2,5-thiadiazol-4-yl)-1-azabicyclo[2 2.1]heptane, (-)-tartrate Mp. 138-140°C. Compound 61.
Endo (β)-3-(3-(3-(3,5-Dichlorophenyl)-2-propyn-1-yloxy)-1,2,5-thiadiazol-4-yl)-1-azabicyclo[2 2.1]heptane, (-)-tartrate. Mp. 103-105°C. Compound 62.
Endo (β)-3-(3-(3-(3,5-Difluorophenyl)-2-propyn-1-yloxy)-1,2,5-thiadiazol-4-yl)-1-azabicyclo[2.2.1]heptane, (-)-tartrate. Mp. 158-160°C. Compound 63.
Endo (β)-3-(3-(3-(3-Bromophenyl)-2-propyn-1-yloxy)-1,2,5-thiadiazol-4-yl)-1-azabicyclo[2.2.1]heptane, (-)-tartrate. Mp. 75-77°C. Compound 64.
Endo (β)-3-(3-(3-(3-Fluorophenyl)-2-propyn-1-yloxy)-1,2,5-thiadiazol-4-yl)-1-azabicyclo[2.2.1)heptane, (-)-tartrate. Mp. 120-122°C. Compound 65.
Endo (β)-3-(3-(3-(3-Chloro-5-fluorophenyl)-2-propyn-1-yloxy)-1,2,5-thiadiazol-4-yl)-1-azabicyclo[2.2.1]heptane, (-)-tartrate. Mp. 101-103°C Compound 66.
Endo (β)-3-(3-(3-(3-Bromo-5-fluorophenyl)-2-propyn-1-yloxy)-1,2,5-thiadiazol-4-yl)-1-azabicyclo[2.2 1]heptane, (-)-tartrate. Mp. 137-138°C. Compound 67
Endo (β)-3-(3-(3-(5-Chloro-2-thienyl)-2-propyn-1-yloxy)-1,2,5-thiadiazol-4-yl)-1-azabicyclo[2.2.1]heptane, (-)-tartrate. Mp 139-141°C. Compound 68.

### Example 4

### Endo (α)-3-(3-(3-Phenyl-2-propyn-1-ylthio)-1.2.5-thiadiazol-4-yl)-1-azabicyclo[2.2 1]heptane. (+)-tartrate.

Endo (+)-3-(3-propylsulfonyl-1,2,5-thiadiazol-4-yl)-1-azabicyclo[2.2.1]heptane (WO 94/20496) (0 4 g, 1.4 mmol) and sodium hydrosulfide monohydrate (0.27 g, 3.8 mmol) were dissolved in dry DMF (50 ml) and the reaction mixture was refluxed at 100 °C for 3 hours (or to completion of reaction). After cooling to 0 °C, potassium carbonate (0 76 g, 5.6 mmol) and 3-phenyl-2-propyn-1-yl methylsulfonate (0.51 g, 2 4 mmol) were added The reaction mixture was stirred at room temperature for 4 hours (or to completion of reaction), after which 1 N hydrogen chloride was added to pH 2 The aqueous phase was first washed with ether (2x50 ml), then potassium carbonate was added to pH>10 and finely the water phase was extracted with ether (2x100 ml). The combined and dried ether phases were evaporated and the residue (0 4 g) was crystallized with L-(+)-tartaric acid (0.2 g, 1.3 mmol) from isopropanol to give the title compound in 0.54 g yield (82 %). Mp. 170-172°C. Compound 12.

The following compounds were made in exactly the same manner using the appropnate methylsulfonate:
Endo (α)-3-(3-(3-(4-Chlorophenyl)-2-propyn-1-ylthio)-1,2,5-thiadiazol-4-yl)-1-azabicyclo[2.2.1]heptane, (+)-tartrate. M p.183-186°C Compound 69.
Endo (a)-3-(3-(3-(3,5-Difluorophenyl)-2-propyn-1-ylthio)-1,2,5-thiadiazol-4-yl)-1-azabicyclo[2.2.1]heptane, (+)-tartrate. M.p.150-151°C Compound 70
Endo (α)-3-(3-(3-(3-Chloro-5-fluorophenyl)-2-propyn-1-ylthio)-1,2,5-thiadiazol-4-yl)-1-azabicyclo[2.2.1]heptane, (+)-tartrate. M.p.118-119°C. Compound 71

### Example 5

### Exo (+-) 3-(3-(3-Phenyl-2-propyn-1-yloxy)-1.2.5-thiadlazol-4-yl)-1-azabicyclo[2 2.1]heptane, (+)-tartrate.

To a mixture of sodium hydride (60% in mineral oil, 0 63 g, 15 mmol) in THF (50ml) was added 3-phenyl-2-propyn-1-ol (0.46 g, 3.5 mmol) at room temperature and the reaction mixture was stirred for 1 hour. After cooling to 0°C, exo (+-) 3-(3-propylsulfonyl-1,2,5-thiadiazol-4-yl)-1-azabicyclo[2.2.1]heptane (WO 94/20496) (0.40 g, 1 4 mmol) was added. The reaction mixture was stirred for 5 hours at room temperature and at 5 °C overnight. 1 N hydrogen chloride (100 ml) was added and the mixture was washed with ether (100 ml). 25% ammonia was added to the water phase to pH >10 and then extracted with ether (2x100 ml). The combined and dried ether phases were evaporated to give an oil (0.39 g). Crystallization with L-(+)-tartanc acid (0.21 g, 1.4 mmol) from isopropanol gave the title compound in 0.51 g (80%) yield Mp.159-162°C. Compound 13.

The following compounds were made in exactly the same manner using the appropriate alcohol:
Exo (+-) 3-(3-(1-(3-Methoxyphenyl)-4-methyl-1-pentyn-3-yloxy)-1,2,5-thiadiazol-4-yl)-1-azabicyclo[2.2 1]heptane, (+)-tartrate. Mp. 134-137°C Compound 14
Exo (+-) 3-(3-(1-(4-Chlorophenyl)-4-methyl-1-pentyn-3-yloxy)-1,2,5-thiadiazol-4-yl)-1-azabicyclo[2.2.1]heptane, (+)-tartrate. Mp. 146-147°C. Compound 15.
Exo (+-) 3-(3-(1-(3- Thienyl)-4-methyl-1-pentyn-3-yloxy)-1 ,2,5-thiadiazol-4-yl)-1-azabicyclo[2.2.1]heptane, (+)-tartrate. Mp. 133-138 °C. Compound 16.

### Example 6

### Exo (+-) 3-(3-(3-(4-Chlorophenyl)-2-propyn-1-ylthio)-1,2,5-thiadiazol-4-yl)-1-azabicyclo[2.2.1]heptane, (+)-tartrate.

Exo (+-) 3-(3-propylsulfonyl-1,2,5-thiadiazol-4-yl)-1-azabicyclo[2.2.1]heptane (WO 94/20496) (0.275 g, 0.95 mmol) and sodium hydrosulfide monohydrate (0.21 g, 2.8 mmol) were dissolved in dry DMF (35 ml) and the reaction mixture was refluxed at 100 °C for 3 hours (or to completion of reaction). After cooling to 0 °C, potassium carbonate (0.5 g, 3.7 mmol) and 3-(4-chlorophenyl)-2-propyn-1-yl methyl sulfonate (0.41 g, 1.67 mmol) were added The reaction mixture was stirred at room temperature for 4 hours (or to completion of reaction), after which 1 N hydrogen chtonde was added to pH 2 The aqueous phase was first washed with ether (2x50 ml), then 25% ammonia was added to pH>10 and finely the water phase was extracted with ether (2x100 ml). The combined and dned ether phases were evaporated and the residue was crystallized with L-(+)-tartanc acid from isopropanol and ether to give the title compound in 40 mg yield Mp. 188-190°C. Compound 17.

### Example 7

### (3S) 3-(3-(3-(4-Chlorophenyl)-2-propyn-1-yloxy)-1,2,5-thiadiazol-4-yl)-1-azabicyclo[2.2.2]octane. (+)-tartrate.

To a mixture of sodium hydride (60% in mineral oil, 0.72 g, 18 mmol) in THF (50 ml) was added 3-(4-chlorophenyl)-2-propyn-1-ol (0.55 g, 3.3 mmol) at 0 °C. The reaction was stirred at 0 °C for 1 hour and then (3S) 3-(3-butylsulfonyl-1,2,5-thiadiazol-4-yl)-1-azabicyclo[2.2 2]octane (Chirality, 1997, in press.) (0.50 g, 1.6 mmol) was added. The reaction mixture was stirred for 18 hours at room temperature. 1 N hydrogen chloride (75 ml) was added and the reaction mixture was washed with ether (2x50 ml). The water phase was made basic (pH>10) with potassium carbonate and then extracted with ether (3x100 ml). The combined ether phases were dried and evaporated. The residue was purified by column chromatography using ethyl acetate/methanol/ 25% ammonia (3:1:1 %) as eluent The product was crystallized with L-(+)-tartaric acid from isopropanol to give the title compound in 310 mg (54%) yield. Mp. 158-163°C Compound 18.

The following compounds were made in exactly the same manner using the appropriate alcohol:
(3S) 3-(3-(3-(3-Chlorophenyl)-2-propyn-1-yloxy)-1,2,5-thiadiazol-4-yl)-1-azabicydo[2.2.2]octane,(+)-tartrate. Mp. 144-146°C. Compound 19.
(3S) 3-(3-(1-(4-Chlorophenyl)-4-methyl-1-pentyn-3-yloxy)-1,2,5-thiadiazol-4-yl)-1-azabicyclo[2.2 2]octane,(+)-tartrate. Mp (75-128)°C Compound 72.
(3S) 3-(3-(1-(3-Chlorophenyl)-4-methyl-1-pentyn-3-yloxy)-1,2,5-thiadiazol-4-yl)-1-azabicyclo[2.2.2]octane,(+)-tartrate. Mp. 65-67°C. Compound 73
(3S) 3-(3-(1-(4-Fluorophenyl)-4-methyl-1-pentyn-3-yloxy)-1,2,5-thiadiazol-4-yl)-1-azabicyclo[2.2.2]octane, (+)-tartrate. Mp. 98-101 °C. Compound 74.
(3S) 3-(3-(3-(3,5-Dichlorophenyl)-2-propyn-1-yloxy)-1,2,5-thiadiazol-4-yl)-1-azabicyclo[2.2.2]octane, (+)-tartrate. Mp. 110-113°C. Compound 75.
(3S) 3-(3-(3-(3-Bromophenyl)-2-propyn-1-yloxy)-1,2,5-thiadiazol-4-yl)-1-azabicyclo[2.2.2]octane, (+)-tartrate. Mp. 144-146°C. Compound 76.
(3S) 3-(3-(3-(3-Trifluoromethoxyphenyl)-2-propyn-1-yloxy)-1,2,5-thiadiazol-4-yl)-1-azabicyclo[2.2.2]octane, (+)-tartrate. Mp. 123-125°C. Compound 77.
(3S) 3-(3-(3-(3,5-Difluorophenyl)-2-propyn-1-yloxy)-1,2,5-thiadiazol-4-yl)-1-azabicyclo[2.2.2]octane, (+)-tartrate. Mp. 115-117°C. Compound 78.
(3S) 3-(3-(3-(3-Chloro-5-fluorophenyl)-2-propyn-1-yloxy)-1,2,5-thiadiazot-4-yl)-1-azabicyclo[2.2.2]octane, (+)-tartrate Mp 116-118°C. Compound 79
(3S) 3-(3-(3-(3-Bromo-5-fluorophenyl)-2-propyn-1-yloxy)-1,2,5-thiadiazol-4-yl)-1-azabicyclo[2.2.2]octane, (+)-tartrate. Mp 134-136°C. Compound 80
(3S) 3-(3-(3-(5-Chloro-2-thienyl)-2-propyn-1-yloxy)-1,2,5-thiadiazol-4-yl)-1-azabicyclo[2.2.2]octane, (+)-tartrate. Mp. 154-156 °C. Compound 81

### Example 8

### Endo (5R,6S)-6-(3-(3-Phenyl-2-propyn-1-ylthio)-1,2,5-thiadiazol-4-yl)-1-azabicyclo[3,2 1]octane, (+)-tartrate

Endo (5R,6S)-3-(3-propylsulfonyl-1,2,5-thiadiazol-4-yl)-1-azabicyclo[3.2.1]octane (WO 94/20496) (0.5 g, 1 66 mmol) and sodium hydrosulfide monohydrate (0 4 g, 5.3 mmol) were dissolved in dry DMF (50 ml) and the reaction mixture was refluxed at 100 °C for 3 hours (or to completion of reaction) After cooling to 0°C potassium carbonate (0.9 g, 6.6 mmol) and 3-phenyl-2-propyn-1-yl methylsulfonate (1 0 g, 4 76 mmol) were added. The reaction mixture was stirred at room temperature for 4 hours (or to completion of reaction), after which 1 N hydrogen chlonde was added to pH 2. The aqueous phase was first washed with ether (2x50 ml), then potassium carbonate was added to pH>10 and finely the water phase was extracted with ether (2x100 ml). The combined and dried ether phases were evaporated and the residue (0 4 g) was crystallized with L-(+)-tartaric acid (0.2 g, 1.3 mmol) from isopropanol to give the title compound in 0.53 g yield (65%). Mp.171-173°C. Compound 20.

The following compounds were made in exactly the same manner using the appropnate methylsulfonate:
Endo (5R,6S)-6-(3-(3-(3-Fluorophenyl)-2-propyn-1-ylthio)-1,2,5-thiadiazol-4-yl)-1-azabicyclo[3.2.1]octane, (+)-tartrate. Mp. 138-140°C. Compound 21
Endo (5R,6S)-6-(3-(3-(4-Fluorophenyl)-2-propyn-1-ylthio)-1,2,5-thiadiazol-4-yl)-1-azabicyclo[3 2.1]octane, (+)-tartrate. Mp. 198-199°C. Compound 22.
Endo (5R,6S)-6-(3-(3-(4-Chlorophenyl)-2-propyn-1-ylthio)-1,2,5-thiadiazol-4-yl)-1-azabicyclo[3.2.1]octane. (+)-tartrate. Mp. 190-192.5°C. Compound 23.
Endo (5R,6S)-6-(3-(3-(3-Thienyl)-2-propyn-1-ylthio)-1,2,5-thiadiazol-4-yl)-1-azabicyclo[3.2.1]octane, (+)-tartrate. Mp. 185-187°C Compound 24
Endo (5R,6S)-6-(3-(3-(2-Thienyl)-2-propyn-1-ylthio)-1,2,5-thiadiazol-4-yl)-1-azabicyclo[3.2.1]octane, (+)-tartrate. Mp. 187-189°C. Compound 25
Endo (5R,6S)-6-(3-(3-(2-Fluorophenyl)-2-propyn-1-ylthio)-1,2,5-thiadiazol-4-yl)-1-azabicyclo[3.2 1]octane, (+)-tartrate. Mp 149-152°C. Compound 82.
Endo (5R,6S)-6-(3-(3-(3-Methylthiophenyl)-2-propyn-1-ylthio)-1,2,5-thiadiazol-4-yl)-1-azabicyclo[3.2 1]octane, (+)-tartrate. Mp. 68-71°C. Compound 83
Endo (5R,6S)-6-(3-(1-(3-Methoxyphenyl)-4-methyl-1-pentyn-3-ylthio)-1,2,5-thiadiazol-4-yl)-1-azabicyclo[3.2.1]octane, (+)-tartrate. Mp. 69-71°C. Compound 84
Endo (5R,6S)-6-(3-(1-(3-Methoxyphenyl)l-1-pentyn-3-ylthio)-1,2,5-thiadiazol-4-yl)-1-azabicyclo[3.2.1]octane, (+)-tartrate. Mp. 63-66°C. Compound 85.
Endo (5R,6S)-6-(3-(1-(2-Thienyl)-4-methyl-1-pentyn-3-ylthio)-1,2,5-thiadiazol-4-yl)-1-azabicyclo[3.2.1]octane, (+)-tartrate. Mp. 68-70°C. Compound 86.
Endo (5R,6S)-6-(3-(3-(3,5-Difluorophenyl)-2-propyn-1-ylthio)-1,2,5-thiadiazol-4-yl)-1-azabicyclo[3.2.1]octane, (+)-tartrate. Mp. 109-111°C Compound 87.

### Example 9

### Endo (5R,6S)-6-(3-(3-Phenyl-2-propyn-1-yloxy)-1,2,5-thiadiazol-4-yl)-1-azabicyclo[3 2 1]octane, (+)-tartrate,

To a mixture of sodium hydride (60% in mineral oil, 0.7 g, 17.5 mmol) in THF (50ml) was added 3-phenyl-2-propyn-1-ol (0.74 g, 5.6 mmol) at room temperature and the reaction mixture was stirred for 1 hour After cooling to 0°C, endo (5R,6S)-6-(3-propylsulfonyl-1,2,5-thiadiazol-4-yl)-1-azabicyclo[3 2.1]octane (WO 94/20496) (0 6 g, 2.0 mmol) was added. The reaction mixture was stirred for 5 hours at room temperature and at 5 °C overnight. Water (50 ml) was added and the mixture was extracted with ether (3x 100 ml). The ether phases were evaporated and the residue was dissolved in 1 N hydrochloric acid (20 ml). The water phase was washed with ether (100 ml), made basic with 25% aqueous ammonia and then extracted with ether (2x100 ml) The ether phases were dried and evaporated to give crude product (0.54 g). Purification on column chromatography eluting with ethyl acetate^{.} methanol:25%aq.NH₃ (2:1 2%) gave the desired free base product as an oil. Crystallization with L-(+)-tartaric acid (0.27 g, 1.8 mmol) from isopropanol gave the title compound in 735 mg (77%) yield. Mp. 163-165 °C. Compound 26.

The following compounds were made in exactly the same manner using the appropnate alcohol:
Endo (5R,6S)-6-(3-(3-(4-Fluorophenyl)-2-propyn-1-yloxy)-1,2,5-thiadiazol-4-yl)-1-azabicyclo[3.2.1]octane, (+)-tartrate. Mp. 147-149 °C. Compound 27.
Endo (5R,6S)- 6-(3-(3-(3-Methoxyphenyl)-2-propyn-1-yloxy)-1,2,5-thiadiazol-4-yl)-1-azabicyclo[3.2.1]octane, (+)-tartrate. Mp. 138-141°C. Compound 28.
Endo (5R,6S)- 6-(3-(3-(4-Chlorophenyl)-2-propyn-1-yloxy)-1,2,5-thiadiazol-4-yl)-1-azabicyclo[3.2.1]octane, (+)-tartrate. Mp 173-174°C. Compound 29.
Endo (5R,6S)- 6-(3-[3-(3-Thienyl)-2-propyn-1-yloxy]-1,2,5-thiadiazol-4-yl)-1-azabicyclo[3.2.1]octane, (+)-tartrate. Mp. 158-160°C. Compound 30.
Endo (5R,6S)-6-(3-[3-(3-Pyridyl)-2-propyn-1-yloxy]-1,2,5-thiadiazol-4-yl)-1-azabicyclo[3.2.1]octane, (+)-tartrate. Mp. 136-139°C. Compound 31.

### Example 10

### Endo (5S,6R)-6-(3-(3-Phenyl-2-propyn-1-yloxy)-1,2,5-thiadiazol-4-yl)-1-azabicyclo[3.2.1]octane, (+)-tartrate,

To a mixture of sodium hydride (60% in mineral oil, 0.6 g, 15 mmol) in THF (50ml) was added 3-phenyl-2-propyn-1-ol (0.62 g, 4.7 mmol) at room temperature and the reaction mixture was stirred for 1 hour. After cooling to 0 °C, endo (5S,6R)-6-(3-propylsulfonyl-1,2,5-thiadiazol-4-yl)-1-azabicyclo[3.2.1]octane (WO 94/20496) (0.5 g, 1.66 mmol) was added. The reaction mixture was stirred for 5 hours at room temperature and at 5 °C overnight. Water (50 ml) was added and the mixture was extracted with ether (3x 100 ml). The ether phases were evaporated and the residue was dissolved in 1 N hydrochlonc acid (20 ml) The water phase was washed with ether (100 ml), made basic with 25% aqueous ammonia and then extracted with ether (2x100 ml). The ether phases were dried and evaporated to give crude product (0.42 g). Purification on column chromatography eluting with ethyl acetate:methanol:25%aq.NH₃ (2^{.}1:2%) gave the desired free base product as an oil. Crystallisation with L-(+) tartaric acid (0.21 g, 1 4 mmol) from isopropanol gave the title compound in 350 mg (44%) yield. Mp. 155-161°C. Compound 32.

The following compounds were made in exactly the same manner using the appropriate alcohol and appropriate acid:
Endo (5S,6R)-6-(3-(3-(4-Fluorophenyl)-2-propyn-1-yloxy)-1,2,5-thiadiazol-4-yl)-1-azabicyclo[3.2.1]octane, (+)-tartrate. Mp. 164-165°C. Compound 33.
Endo (5S,6R)-6-(3-[3-(3-Methylthiophenyl)-2-propyn-1-yloxy]-1,2,5-thiadiazol-4-yl)-1-azabicyclo[3.2.1]octane, (+)-tartrate. Mp. 77-79 °C. Compound 34
Endo (5S,6R)-6-(3-[1-(3-Methoxyphenyl)-4-methyl-1-pentyn-3-yloxy]-1,2,5-thiadiazol-4-yl)-1-azabicyclo[3.2.1]octane, (+)-tartrate. Mp. 72-74 °C. Compound 35.
Endo (5S,6R)-6-(3-[3-(2,5-Difluorophenyl)-2-propyn-1-yloxy]-1,2,5-thiadiazol-4-yl)-1-azabicyclo[3.2.1]octane, (-)-tartrate. Mp. 167-169°C. Compound 36.
Endo (5S,6R)-6-(3-[3-(3,5-Difluorophenyl)-2-propyn-1-yloxy]-1,2,5-thiadiazol-4-yl)-1-azabicyclo[3 2.1]octane. (-)-tartrate. Mp. 154-156 °C Compound 37.
Endo (5S,6R)-6-(3-[1-(2-Thienyl)-4-methyl-1-pentyn-3-yloxy]-1,2,5-thiadiazol-4-yl)-1-azabicyclo[3 2.1]octane, (+)-tartrate. Mp 80-82°C. Compound 38

### Example 11

### Exo (5R,6R)-6-(3-(3-Phenyl-2-propyn-1-ylthio)-1,2,5-thiadiazol-4-yl)-1-azabicyclo[3.2.1]octane. (+)-tartrate.

Exo (5R,6R)-3-(3-propylsulfonyl-1,2,5-thiadiazol-4-y1)-1-azabicyclo[3.2 1]octane (WO 94/20496) (0.43 g, 1.43 mmol) and sodium hydrosulfide monohydrate (0.28 g, 3.7 mmol) were dissolved in dry DMF (50 ml) and the reaction mixture was refluxed at 100°C for 3 hours (or to completion of reaction). After cooling to 0°C potassium carbonate (0.8 g, 5.7 mmol) and 3-phenyl-2-propyn-1-yl methylsulfonate (0.5 g, 2 5 mmol) were added. The reaction mixture was stirred at 0°C for 4 hours (or to completion of reaction), after which 1 N hydrogen chloride was added to pH 2. The aqueous phase was first washed with ether (2x50 ml), then potassium carbonate was added to pH>10 and finely the water phase was extracted with ether (2x100 ml). The combined and dried ether phases were evaporated and the residue (0.22 g) was crystallized with L-(+)-tartaric acid (0.1 g, 0.7 mmol) from isopropanol to give the title compound in 0.29 g yield (41%). Mp. 192-192.5°C. Compound 39

The following compounds were made in exactly the same manner using the appropriate methytsutfonate:
Exo (5R,6R)-6-(3-(1-(3-Chlorophenyl)-4-methyl-1-pentyn-3-ylthio)-1,2,5-thiadiazol-4-yl)-1-azabicyclo[3.2.1]octane, (+)-tartrate. Mp.134-136°C. Compound 88.
Exo (5R,6R)-6-(3-(1-(3,5-Difluorophenyl)-4-methyl-1-pentyn-3-ylthio)-1,2,5-thiadiazol-4-yl)-1-azabicyclo[3.2.1]octane, (+)-tartrate. Mp.137-139°C. Compound 89.
Exo (5R,6R)-6-(3-(1-(3-Cyanophenyl)-4-methyl-1-pentyn-3-ylthio)-1,2,5-thiadiazol-4-yl)-1-azabicyclo[3.2 1]octane. (+)-tartrate. Mp.125-126°C. Compound 90
Exo (5R,6R)-6-(3-(3-(3-Bromophenyl)-2-propyn-1-ylthio)-1,2,5-thiadiazol-4-yl)-1-azabicyclo[3.2.1]octane, (+)-tartrate. Mp.193-194°C. Compound 91.
Exo (5R,6R)-6-(3-(3-(3-Chloro-5-fluorophenyl)-2-propyn-1-ylthio)-1,2,5-thiadiazol-4-yl)-1-azabicyclo[3 2.1]octane, (+)-tartrate. Mp.166-168°C Compound 92.
Exo (5R,6R)-6-(3-(3-(3-Bromo-5-fluorophenyl)-2-propyn-1-ylthio)-1,2,5-thiadiazol-4-yl)-1-azabicyclo[3.2.1]octane, (+)-tartrate. Mp.167-169°C. Compound 93.
Exo (5R,6R)-6-(3-(3-(5-Pyrimidyl)-2-propyn-1-ylthio)-1,2,5-thiadiazol-4-yl)-1-azabicyclo[3.2.1]octane, (+)-tartrate. Mp.147-148°C. Compound 94.

### Example 12

### Exo (5R,6R)-6-(3-(3-Phenyl-2-propyn-1-yloxy)-1,2,5-thiadiazol-4-yl)-1-azabicyclo[3.2.1]octane, oxalate.

To a mixture of sodium hydride (60% in mineral oil, 0.6 g, 15 mmol) in THF (50ml) was added 3-phenyl-2-propyn-1-ol (0.62 g, 4.7 mmol) at room temperature and the reaction mixture was stirred for 1 hour. After cooling to 0 °C, exo (5R,6R) 6-(3-propylsulfonyl-1,2,5-thiadiazol-4-yl)-1-azabicyclo[3.2.1]octane (WO 94/20496) (0.5 g, 1.66 mmol) was added. The reaction mixture was stirred for 5 hours at room temperature and at 5°C overnight. Water (50 ml) was added and the mixture was extracted with ether (3x 100 ml). The ether phases were evaporated and the residue was dissolved in 1 N hydrochloric acid (20 ml). The water phase was washed with ether (100 ml), made basic with 25% aqueous ammoma and then extracted with ether (2x100 ml). The ether phases were dried and evaporated to give crude product. Purification on column chromatography eluting with ethyl acetate:methanol.25%aq.NH₃ (2:1^{.}2%) gave the desired free base product as an oil. Crystallisation with oxalic acid gave the title compound in 560 mg (78%) yeld Mp 120-125°C. Compound 40.

The following compounds were made in the same manner using the appropriate alcohol and appropnate acid:
Exo (5R,6R)-6-(3-(3-(3-Methoxyphenyl)-2-propyn-1-yfoxy)-1,2,5-thiadiazol-4-yl)-1-azabicyclo[3.2.1]octane, oxalate. Mp. 116-117.5°C. Compound 41.
Exo (5R,6R)-6-(3-(3-(3-Trifluoromethylphenyl)-2-propyn-1-yloxy)-1,2,5-thiadiazol-4-yl)-1-azabicyclo[3.2.1]octane, (+)-tartrate. Mp 109-115°C. Compound 42.
Exo (5R,6R)-6-(3-(3-(3-Methoxyphenyl)-1-methyl-2-propyn-1-yloxy)-1,2,5-thiadiazol-4-yl)-1-azabicyclo[3.2.1]octane, (+)-tartrate. Mp. 134.5-136.5°C. Compound 43.
Exo (5R,6R)-6-(3-(3-(4-Chlorophenyl)-2-propyn-1-yloxy)-1,2,5-thiadiazol-4-yl)-1-azabicyclo[3.2.1]octane, (+)-tartrate. Mp. 188-190°C. Compound 44
Exo (5R,6R)-6-(3-(1-(3-Methoxyphenyl)-4-methyl-1-pentyn-3-yloxy)-1,2,5-thiadiazol-4-yl)-1-azabicyclo[3 2.1]octane, (+)-tartrate. Mp. 153-156°C. Compound 45.
Exo (5R,6R)-6-(3-(1-(4-Chlorophenyl)-4-methyl-1-pentyn-3-yloxy)-1,2,5-thiadiazol-4-yl)-1-azabicyclo[3 2.1]octane, (+)-tartrate. Mp. 154-157.5°C. Compound 46
Exo (5R,6R)-6-(3-(3-(2-Thienyl)-1-methyl-2-propyn-1-yloxy)-1,2,5-thiadiazol-4-yl)-1-azabicyclo[3.2.1]octane, (+)-tartrate. Mp 150-151°C. Compound 47.
Exo (5R,6R)-6-(3-(3-(3-Thienyl)-1-methyl-2-propyn-1-yloxy)-1,2,5-thiadiazol-4-yl)-1-azabicyclo[3.2.1]octane, (+)-tartrate Mp. 146.5-147.5°C. Compound 48.
Exo (5R,6R)-6-(3-[3-(2-Thienyl)-2-propyn-1-yloxy]-1,2,5-thiadiazol-4-yl)-1-azabicyclo[3 2 1]octane, oxalate. Mp. 137-139°C. Compound 49.
Exo (5R,6R)-6-(3-[3-(3-Thienyl)-2-propyn-1-yloxy]-1,2,5-thiadiazol-4-yl)-1-azabicyclo[3.2.1]octane, oxalate. Mp. 131-132°C. Compound 50.
Exo (5R,6R)-6-(3-(1-(3-Thienyl)-4-methyl-1-pentyn-3-yloxy)-1,2,5-thiadiazol-4-yl)-1-azabicyclo[3.2.1]octane, (+)-tartrate. Mp. 145-148°C. Compound 51.
Exo (5R,6R)-6-(3-(4-methyl-1-phenyl-1-pentyn-3-yloxy)-1,2,5-thiadiazol-4-yl)-1-azabicyclo[3.2.1]octane, (+)-tartrate. Mp. 146-148°C. Compound 95.
Exo (5R,6R)-6-(3-(1-(3,5-Dichlorophenyl)-4-methyl-1-pentyn-3-yloxy)-1,2,5-thiadiazol-4-yl)-1-azabicyclo[3.2.1]octane, (+)-tartrate Mp. 180-182°C. Compound 96.
Exo (5R,6R)-6-(3-(3-(3-Cyanophenyl)-2-propyn-1-yloxy)-1,2,5-thiadiazol-4-yl)-1-azabicyclo[3.2. 1]octane, (+)-tartrate. Mp. 136-138°C. Compound 97.
Exo (5R,6R)-6-(3-(1-(3-Chlorophenyl)-4-methyl-1-pentyn-3-yloxy)-1,2,5-thiadiazol-4-yl)-1-azabicyclo[3 2.1]octane, (+)-tartrate. Mp. 157-159°C. Compound 98.
Exo (5R.6R)-6-(3-(1-(3-Cyanophenyl)-4-methyl-1-pentyn-3-yloxy)-1,2,5-thiadiazol-4-yl)-1-azabicyclo[3.2.1]octane, (+)-tartrate. Mp 160-162°C. Compound 99.
Exo (5R,6R)-6-(3-(1-(3,5-Difluorophenyl)-4-methyl-1-pentyn-3-yloxy)-1,2,5-thiadiazol-4-yl)-1-azabicyclo[3 2.1]octane, (+)-tartrate. Mp. 185-187°C. Compound 100
Exo (5R,6R)-6-(3-(3-(3,5-Difluorophenyl)-2-propyn-1-yloxy)-1,2,5-thiadiazol-4-yl)-1-azabicyclo[3.2.1]octane, (+)-tartrate. Mp. 138-140°C. Compound 101.
Exo (5R,6R)-6-(3-(3-(3-Bromophenyl)-2-propyn-1-yloxy)-1,2,5-thiadiazol-4-yl)-1-azabicyclo[3 2.1]octane, (+)-tartrate Mp 171-173°C. Compound 102.
Exo (5R,6R)-6-(3-(3-(3-Chloro-5-fluorophenyl)-2-propyn-1-yloxy)-1,2,5-thiadiazol-4-yl)-1-azabicyclo[3.2.1]octane, (+)-tartrate. Mp 167-168°C. Compound 103.
Exo (5R,6R)-6-(3-(3-(3-Bromo-5-fluorophenyl)-2-propyn-1-yloxy)-1,2,5-thiadiazof-4-yl)-1-azabicyclo[3.2.1]octane, (+)-tartrate. Mp. 162-163°C. Compound 104.
Exo (5R,6R)-6-(3-(1-(2-Thienyl)-4-methyl-1-pentyn-3-yloxy)-1,2,5-thiadiazol-4-yl)-1-azabicyclo[3.2.1]octane, (+)-tartrate. Mp. 135-137°C Compound 105
Exo (5R,6R)-6-(3-(3-(3-Furyl)-2-propyn-1-yloxy)-1,2,5-thiadiazol-4-yl)-1-azabicyclo[3.2.1]octane, (+)-tartrate. Mp. 160-161°C. Compound 106.

### Example 13

### Exo (5S,6S)-6-(3-(3-Phenyl-2-propyn-1-yloxy)-1,2,5-thiadiazol-4-yl)-1-azabicyclo[3.2.1]octane, oxalate

To a mixture of sodium hydride (60% in mineral oil, 0.6 g, 15 mmol) in THF (50ml) was added 3-phenyl-2-propyn-1-ol (0.62 g, 4 7 mmol) at room temperature and the reaction mixture was stirred for 1 hour. After cooling to 0°C. exo (5S,6S)-6-(3-propylsulfonyl-1,2,5-thiadiazol-4-yl)-1-azabicyclo[3.2.1]octane (WO 94/20496) (0.5 g, 1.66 mmol) was added The reaction mixture was stirred for 5 hours at room temperature and at 5°C overnight. Water (50 ml) was added and the mixture was extracted with ether (3x100 ml). The ether phases were evaporated and the residue was dissolved in 1 N hydrochloric acid (20 ml). The water phase was washed with ether (100 ml), made basic with 25% aqueous ammonia and then extracted with ether (2x100 ml). The ether phases were dried and evaporated to give crude product. Purification on column chromatography eluting with ethyl acetate:methanoi:25%aq.NH₃ (2.1:2%) gave the desired free base product as an oil Crystallisation with oxalic acid gave the title compound in 520 mg (72%) yield Mp.132-135°C. Compound 52.

### Example 14

### (+-)3-(3-(3-Phenyl-2-propyn-1-yloxy)-1,2,5-thiadiazol-4-yl)-1-azabicyclo[2.2.2]octane, oxalate.

To a solution of 3-phenyl-2-propyn-1-ol (3 0 g, 15 mmol) in THF (20 ml) was added sodium hydnde (80% in mineral oil, 0.45 g, 15 mmol). The reaction mixture was stirred at room temperature for 1 hour and then a solution of (+-) 3-(3-chloro-1,2,5-thiadiazol-4-yl)-1-azabicyclo[2.2.2]octane *(Eur J Med. Chem.*,**1996*****,** 31*, 221-230) (1.15 g, 5 mmol) in THF (15 ml) was added The reaction mixture was stirred at 50°C for 18 hours. 1 N hydrogen chloride was added and the THF phase was discharged. The water phase was made basic with sodium hydroxide and then extracted with ether (3x100 ml). The combined ether phases were dried and evaporated to give crude product The residue was punfied by column chromatography eluting with ethyl acetate^{.}methanol:25%aq.NH₃ (80,20:0.5) Crystallisation with oxalic acid from acetone gave the title compound in 310 mg (15%) yield. Mp. 91-94°C. Compound 53.

The following compound was made in exactly the same manner using the appropriate alcohol:
(+-) 3-(3-(3-(4-Fluorophenyl)-2-propyn-1-yloxy)-1,2,5-thiadiazol-4-yl)-1-azabicyclo[2.2.2]octane, oxalate. Mp. 102-103°C. Compound 54.

### Example 15

### (+-)3-(3-(3-(4-Chlorophenyl)-2-propyn-1-ylthio)-1,2,5-thiadiazol-4-yl)-1-azabicyclo[2.2.2]octane, (+)-tartrate.

To a solution of (+-) 3-(3-chloro-1,2,5-thiadiazol-4-yl)-1-azabicyclo[2.2.2]octane *(Eur. J. Med. Chem.,* **1996,** *31*, 221-230) (0.8 g, 3.5 mmol) in DMF (20 ml) was added sodium hydrosulfide monohydrate (0.66 g, 8 mmol). The reaction mixture was stirred at room temperature for 5 hours. Potassium carbonate (1.38 g, 10 mmol) and 3-(4-chlorophenyl)-2-propyn-1-yl methylsulfonate (0.87 g, 3 5 mmol) was added. The reaction mixture was stirred at room temperature over night. 1 N hydrochloric acid was added and the mixture was washed with ether (100 ml). The water phase was made basic with potassium carbonate and extracted with ether (3x100 ml). The combined ether phases were dried and evaporated to give crude product (1.22 g). Crystallisation with L-(+)tartaric acid from isopropanol followed by recrystallisation from acetone-isopropanol gave the title compound in 960 mg (52%) yield. Mp. 184-185°C. Compound 55.

The following compound was made in the same manner using the appropriate methylsulfonate:
(+-)3-(3-(3-(3-Chlorophenyl)-2-propyn-1-ylthio)-1,2.5-thiadiazol-4-y))-1-azabicyclo[2 2.2]octane, (+)-tartrate. Mp. 168-170°C. Compound 111.

### Example 16

### Endo (β) 3-(3-( 3-(3,5-Difluorophenyl)-2-propyn-1-ylthio)-1.2.5-thiadiazol-4-yl)-1-azabicyclo[2.2.1]heptane, (-)-tartrate

Endo (-) 3-(3-{3-propylsulfonyl-1,2,5-thiadiazol-4-yl)-1-azabicyclo[2.2.1]heptane (WO94/20496) (1 5 g, 5.2 mmol) and potassium carbonate (1.4 g, 10.4 mmol) in dry DMF(20 ml) was added sodium hydrosulfide monohydrate (1.15 g, 15 6 mmol). The reaction mixture was heated to 70°C Dunng this period another two portions of sodium hydrosulfide monohydrate was added to complete the reaction. DMF was evaporated and the residue added ice water followed by 4 N hydrochloric acid to pH 8-9 The product was isolated by filtration to give 862 mg (78%) of Endo (β)-4-(1-azabicyclo[2.2.1]hept-3-yl)-1,2,5-thiadiazol-3-thiol.

To a suspension of endo (β)-4-(1-azabicyclo[2.2.1]hept-3-yl)-1,2,5-thiadiazol-3-thiol (107 mg, 0.5 mmol) and potassium carbonate (123 mg, 0.5 mmol) in dry THF (10 ml) cooled on ice was under nitrogen added a solution of 3-(3,5-difluorophenyl)-2-propyn-1-yl methylsulfonate (123 mg, 0.5 mmol) in dry THF (5 ml). The reaction mixture was stirred overnight and the temperature allowed to raise to room temperature Ice water was added and pH adjusted with 1 N hydrochloric acid to pH 2.0. THF was evaporated and the residue washed with ether (3 x 50 ml). The water phase was made basic with 25% aqueous ammonia (pH 10-11) and the product extracted with ether (3 x 50 ml). The dried ether phases were evaporated and the residue crystallised with D-(-)-tartaric acid in isopropanole. The title compound was isolated by filtration in 100 mg yield (40 %). Mp 142-144°C. Compound 107.

The following compound was made in the same manner using the appropnate methylsulfonate:
Endo (β) 3-(3-(3-(3-Fluorophenyl)-2-propyn-1-ylthio)-1,2,5-thiadiazol-4-yl)-1-azabicyclo[2.2.1]heptane, (-)-tartrate. Mp. 170-171°C. Compound 108.

### Example 17

### Endo (β)3-(3-(3-(3-Chloro-5-fluorophenyl)-2-propyn-1-ylthio)-1,2,5-thiadiazol-4-yl)-1-azabicyclo[2 2.1]heptane, (-)-tartrate

To an ice cooled suspension of endo (β)-4-(1-azabicyclo[2.2 1]hept-3-yl)-1,2,5-thiadiazol-3-thiol (described in example 16) (213 mg, 1.0 mmol) and triphenylphosphine (262 mg, 1 0 mmol) in dry THF (10 ml) was under nitrogen added 3-(3-chloro-5-fluorophenyl)-prop-2-yn-1-ol (277 mg, 1.5 mmol) in THF (5 ml). Diethyl azodicarboxylate (174 mg, 1.0 mmol) was added and the temperature allowed to raise to room temperature while stirring overnight. Ice water was added and pH adjusted with 4 N hydrochloric acid (pH 2.0). THF was evaporated and the water phase washed with ether (2 x 75 ml). 25 % Aqueous ammonia was added (pH 10-11) and the product extracted by ether (3 x 75 ml) The dried ether phases were evaporated and the residue crystallised with D-(-)-tartaric acid in isopropanole. The title compound was isolated by filtration in 245 mg yield (46 %). Mp. 68-70°C. Compound 109.

The following compound was made in the same manner using the appropriate alcohol:
Endo (β) 3-(3-(3-(3-Bromo-5-fluorophenyl)-2-propyn-1-ylthio)-1,2,5-thiadiazol-4-yl)-1-azabicyclo[2.2.1]heptane, (-)-tartrate. Mp. 81-82°C. Compound 110

### Example 18

### (3S) 3-(3-(3-(3-Fluorophenyl)-2-propyn-1-ylthio)-1,2,5-thiadiazol-4-yl)-1-azabicyclo[2.2.2]octane, (+)-tartrate

To a solution of (3S) 3-(3-butylsulfonyl-1,2,5-thiadiazol-4-yl)-1-azabicyclo[2.2 2]octane (Chirality, 1997, in press) (1.90 g, 6.0 mmol) in dry DMF (15 ml) was added sodium hydrosulfide monohydrate (1.67 g, 18.0 mmol) and the mixture was stirred at 95°C for 3 h The reaction mixture was concentrated in vacuo, added ice water and made less basic with 4 N hydrachloride acid The precipitate was isolated by filtration to give 1.05 g (77%) of (3S) 3-(3-mercapto-1,2,5-thiadiazol-4-yl)-1-azabicyclo[2.2.2]octane

To an ice cooled suspension of (3S) 3-(3-mercapto-1,2,5-thiadiazol-4-yl)-1-azabicyclo[2.2 2]octane (340 mg, 1 5 mmol) and triphenylphosphine (390 mg, 1 5 mmol) in dry THF (30 ml) was under nitrogen added 3-(4-fluorophenyl)-prop-2-yn-1-ol (350 mg, 2.3 mmol) in dry THF (25 ml) followed by diethyl azodicarboxylate (260 mg, 1.5 mmol). After stirring at room temperature for 48 h the mixture was concentrated in vacuo and the residue submitted to flash chromatography using methanol/ethyl acetate (1 4) graduated to methanol/ethyl acetate/ammonium hydroxide (1:3:3%) as the eluent. The purified product was crystallised with L-(+)-tartanc acid in isopropanol to give 440 mg (86%) of the title compound. Mp. 174-175°C. Compound 112.

The following compounds were made in the same manner using the appropriate alcohol:
(3S) 3-(3-(3-(3,5-Dichlorophenyl)-2-propyn-1-ylthio)-1,2,5-thiadiazol-4-yl)-1-azabicyclo[2.2.2]octane, (+)-tartrate. Mp 103-105°C. Compound 113.
(3S) 3-(3-(3-(3-Bromophenyl)-2-propyn-1-ylthio)-1,2,5-thiadiazol-4-yl)-1-azabicyclo[2.2.2]octane, (+)-tartrate. Mp. 175-176°C. Compound 114.
(3S) 3-(3-(3-(3-Trifluoromethoxyphenyl)-2-propyn-1-ylthio)-1,2,5-thiadiazol-4-yl)-1-azabicyclo[2.2.2]octane, (+)-tartrate. Mp. 153-155°C Compound 115
(3S) 3-(3-(1-(3,5-Difluorophenyl)-4-methyl-1-pentyn-3-ylthio)-1,2,5-thiadiazol-4-yl)-1-azabicyclo[2.2.2]octane, (+)-tartrate. Mp. 146-148°C. Compound 116.
(3S) 3-(3-(3-(3,5-Difluorophenyl)-2-propyn-1-ylthio)-1,2,5-thiadiazol-4-yl)-1-azabicyclo[2.2.2]octane, (+)-tartrate. Mp. 130-133°C. Compound 117
(3S) 3-(3-(3-(3-Bromo-5-fluorophenyl)-2-propyn-1-ylthio)-1,2,5-thiadiazol-4-yl)-1-azabicyclo[2.2.2]octane, (+)-tartrate Mp. 113-115°C. Compound 118
(3S) 3-(3-(3-(5-Chloro-2-thienyl)-2-propyn-1-ylthio)-1,2,5-thiadiazol-4-yl)-1-azabicyclo[2.2.2]octane, (+)-tartrate. Mp. 164-167°C. Compound 119.

## Claims

1. A compound of formula I 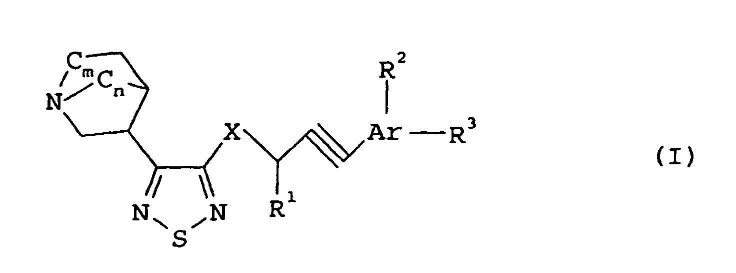 wherein
X is oxygen or sulphur; and
R¹ is hydrogen, straight or branched C₁₋₅-alkyl, straight or branched C₂₋₅-alkenyl, straight or branched C₂₋₅-alkynyl or straight or branched C₄₋₅-alkenynyl, each of which is optionally substituted with one or more halogen(s); and
R² and R³ are independently hydrogen, halogen, CN, NO₂, CF₃, OCF₃, C₁₋₃-alkyl, C₁-₃-alkoxy or C₁₋₃-alkylthio, wherein C₁₋₃-alkyl, C₁₋₃-alkoxy and C₁₋₃-alkylthio are optionally substituted with one or more halogen(s), cyano, amino or nitro; and
Ar is phenyl, thienyl, pyridyl, pyrimidinyl, thiazolyl or furyl; and
n is 0, 1 or 2; and
m is 0, 1 or 2; or
a pharmaceutically acceptable salt or solvate thereof.

2. A compound of formula I 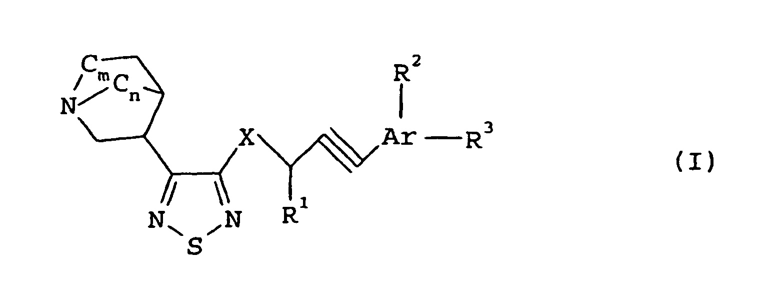 wherein
X is oxygen or sulphur; and
R¹ is hydrogen, straight or branched C₁₋₅-alkyl, straight or branched C₂₋₅-alkenyl, straight or branched C₂₋₅-alkynyl or straight or branched C₄₋₅-alkenynyl, each of which is optionally substituted with one or more halogen(s); and
R² and R³ are independently hydrogen, halogen, CN, NO₂, C₁₋₃-alkyl, C₁₋₃-alkoxy or C₁₋₃-alkylthio, wherein C₁₋₃-alkyl, C₁₋₃-alkoxy and C₁₋₃-alkylthio are optionally substituted with one or more halogen(s), cyano, amino or nitro; and
Ar is phenyl, thienyl, pyridyl, pyrimidinyl, thiazolyl or furyl; and
n is 0, 1 or 2; and
m is 0, 1 or 2; or
a pharmaceutically acceptable salt or solvate thereof.

3. A compound according to claim 1 or 2 wherein X is oxygen.

4. A compound according to claim 1 or 2 wherein X is sulfur.

5. A compound according to claims 1 to 4 wherein R¹ is hydrogen.

6. A compound according to claims 1 to 4 wherein R¹ is straight or branched C₁₋₅-alkyl.

7. A compound according to claims 1 to 4 and 6 wherein R¹ is straight or branched C₁₋₃-alkyl.

8. A compound according to claims 1 to 4 and 6 and 7 wherein R¹ is isopropyl.

9. A compound according to anyone of the preceding claims wherein R² and R³ are halogen(s).

10. A compound according to anyone of the preceding claims wherein Ar is phenyl or thienyl.

11. A compound according to anyone of the preceding claims wherein m is 1 or 2.

12. A compound according to anyone of the preceding claims wherein n is 1 or 2.

13. A compound according to claim 1 which is selected from the following:
Endo (+-) 3-(3-(3-Phenyl-2-propyn-1-yloxy)-1,2,5-thiadiazol-4-yl)-1-azabicyclo[2.2.1]heptane,
Endo (+-) 3-(3-(3-(4-Fluorophenyl)-2-propyn-1-yloxy)-1,2,5-thiadiazol-4-yl)-1-azabicyclo[2.2.1]heptane,
Endo (α) 3-(3-(3-(4-Fluorophenyl)-2-propyn-1-yloxy-1,2,5-thiadiazol-4-yl)-1-azabicyclo[2.2.1]heptane,
Endo (α) 3-(3-(3-Phenyl-2-propyn-1-yloxy)-1 ,2.5-thiadiazol-4-yl)-1-azabicyclo[2.2.1]heptane,
Endo (α) 3-(3-(3-(3-Methoxyphenyl)-2-propyn-1-yloxy)-1 ,2,5-thiadiazol-4-yl)-1-azabicyclo[2.2.1]heptane,
Endo (α) 3-(3-(3-(4-Chlorophenyl)-2-propyn-1-yloxy)-1,2,5-thiadiazol-4-yl)-1-azabicyclo[2.2.1]heptane,
Endo (a) 3-(3-[3-(3-Thienyl)-2-propyn-1-yloxy]-1,2,5-thiadiazol-4-yl)-1-azabicyclo[2.2.1]heptane,
Endo (α) 3-(3-(3-(2-Thienyl)-2-propyn-1-yloxy)-1,2,5-thiadiazol-4-yl)-1-azabicyclo[2.2.1]heptane,
Endo (β) 3-(3-(3-(4-Fluorophenyl)-2-propyn-1-yloxy)-1,2,5-thiadiazol-4-yl)-1-azabicyclo[2.2.1]heptane,
Endo (β) 3-(3-(3-(3-Methoxyphenyl)-2-propyn-1-yloxy)-1,2,5-thiadiazol-4-yl)-1-azabicyclo[2.2.1]heptane,
Endo (β) 3-(3-(3-Phenyl-2-propyn-1-yloxy)-1,2,5-thiadiazol-4-yl)-1-azabicyclo[2.2.1]heptane,
Endo (α) 3-(3-(3-Phenyl-2-propyn-1-ylthio)-1 ,2 ,5-thiadiazol-4-yl)-1-azabicyclo[2.2.1]heptane,
Exo (+-) 3-(3-(3-Phenyl-2-propyn-1-yloxy)-1,2,5-thiadiazol-4-yl)-1-azabicyclo[2.2.1]heptane,
Exo (+-) 3-(3-(1-(3-Methoxyphenyl)-4-methyl-1-pentyn-3-yloxy)-1,2,5-thiadiazol-4-yl)-1-azabicyclo[2.2.1]heptane,
Exo (+-) 3-(3-(1-(4-Chlorophenyl)-4-methyl-1-pentyn-3-yloxy)-1 ,2,5-thiadiazol-4-yl)-1-azabicyclo[2.2.1]heptane,
Exo (+-) 3-(3-(1-(3-Thienyl)-4-methyl-1-pentyn-3-yloxy)-1,2,5-thiadiazol-4-yl)-1-azabicyclo[2.2.1]heptane,
Exo (+-) 3-(3-(3-(4-Chlorophenyl)-2-propyn-1-ylthio)-1,2,5-thiadiazol-4-yl)-1-azabicyclo[2.2.1]heptane,
(3S) 3-(3-(3-(4-Chlorophenyl)-2-propyn-1-yloxy)-1,2,5-thiadiazol-4-yl)-1-azabicyclo[2.2.2]octane,
(3S) 3-(3-(3-(3-Chlorophenyl)-2-propyn-1-yloxy)-1,2,5-thiadiazol-4-yl)-1-azabicyclo[2.2.2]octane,
Endo (5R,6S)-6-(3-(3-Phenyl-2-propyn-1-ylthio)-1,2,5-thiadiazol-4-yl)-1-azabicyclo[3.2.1]octane,
Endo (5R,6S)-6-(3-(3-(3-Fluorophenyl)-2-propyn-1-ylthio)-1,2,5-thiadiazol-4-yl)-1-azabicydo[3.2. 1]octane,
Endo (5R,6S)-6-(3-(3-(4-Fluorophenyl)-2-propyn-1-ylthio)-1,2,5-thiadiazol-4-yl)-1-azabicyclo[3.2.1]octane,
Endo (5R,6S)-6-(3-(3-(4-Chlorophenyl)-2-propyn-1-ylthio)-1,2,5-thiadiazol-4-yl)-1-azabicyclo[3.2.1]octane,
Endo (5R,6S)-6-(3-(3-(3-Thienyl)-2-propyn-1-ylthio)-1,2,5-thiadiazol-4-yl)-1-azabicyclo[3.2.1]octane,
Endo (5R,6S)-6-(3-(3-(2-Thienyl)-2-propyn-1-ylthio)-1,2,5-thiadiazol-4-yl)-1-azabicyclo[3.2.1]octane,
Endo (5R,6S)-6-(3-(3-Phenyl-2-propyn-1-ytoxyr1,2,5-thiadiazol-4-yl)-1-azabicyclo[3.2.1]octane,
Endo (5R,6S)-6-(3-(3-(4-Fluorophenyl)-2-propyn-1-yloxy)-1,2,5-thiadiazol-4-yl)-1-azabicyclo[3.2.1]octane,
Endo (5R,6S)-6-(3-(3-(3-Methoxyphenyl)-2-propyn-1-yloxy)-1,2,5-thiadiazol-4-yl)-1-azabicyclo[3.2.1]octane,
Endo (5R,6S)-6-(3-(3-(4-Chlorophenyl)-2-propyn-1-yloxy)-1,2,5-thiadiazol-4-yi)-i-azabicyclo[3.2.1]octane,
Endo (5R,6S)-6-(3-[3-(3-Thienyl)-2-propyn-1-yloxy]-1,2,5-thiadiazol-4-yl)-1-azabicyclo[3.2.1]octane,
Endo (5R,6S)-6-(3-[3-(3-Pyridyl)-2-propyn-1-yloxy]-1,2,5-thiadiazol-4-yl)-1-azabicyclo[3.2.1]octane,
Endo (5S,6R)-6-(3-(3-Phenyl-2-propyn-1-yloxy)-1,2,5-thiadiazol-4-yl)-1-azabicyclo[3.2.1]octane,
Endo (5S,6R)-6-(3-(3-(4-Fluorophenyl)-2-propyn-1-yloxy)-1 ,2.5-thiadiazol-4-yl)-1-azabicyclo[3.2.1]octane,
Endo (5S,6R)-6-(3-[3-(3-Methylthiophenyl)-2-propyn-1-yloxyl-1,2,5-thiadiazol-4-yl)-1-azabicyclo[3.2.1]octane.
Endo (5S,6R)-6-(3-[1-(3-Methoxyphenyl)-4-methyl-1-pentyn-3-yloxy]-1,2,5-thiadiazol-4-yl)-1-azabicyclo[3.2.1]octane,
Endo (5S,6R)-6-(3-[3-(2,5-Difluorophenyl)-2-propyn-1-yloxy]-1,2,5-thiadiazol-4-yl)-1-azabicyclo[3.2.1]octane,
Endo (5S,6R)-6-(3-[3-(3,5-Difluorophenyl)-2-propyn-1-yloxy]-1,2,5-thiadiazol-4-yl)-1-azabicyclo[3.2.1]octane,
Endo (5S,6R)-6-(3-[1-(2-Thienyl)-4-methyl-1-pentyny-3-yloxy]-1,2,5-thiadiazol-4-yl)-1-azabicyclo[3.2.1]octane,
Exo (5R,6R)-6-(3-(3-Phenyl-2-propyn-1-ylthio)-1,2,5-thiadiazol-4-yl)-1-azabicyclo[3.2.1]octane,
Exo (5R,6R)-6-(3-(3-Phenyl-2-propyn-1-yloxy)-1,2,5-thiadiazol-4-yl)-1-azabicyclo[3.2.1]octane,
Exo (5R,6R)-6-(3-(3-(3-Methoxyphenyl)-2-propyn-1-yloxy)-1,2,5-thiadiazol-4-yl)-1-azabicyclo[3.2.1]octane,
Exo (5R,6R)-6-(3-(3-(3-Trifluoromethylphenyl)-2-propyn-1-yloxy)-1,2,5-thiadiazol-4-yl)-1-azabicyclo[3.2.1]octane,
Exo (5R,6R)-6-(3-(3-(3-Methoxyphenyl)-1-methyl-2-propyn-1-yloxy)-1,2,5-thiadiazol-4-yl)-1-azabicyclo[3.2.1]octane,
Exo (5R,6R)-6-(3-(3-(4-Chlorophenyl)-2-propyn-1-yloxy)-1,2,5-thiadiazol-4-yl)-1-azabicyclo[3.2.1]octane,
Exo (5R,6R)-6-(3-(1-(3-Methoxyphenyl)-4-methyl-1-pentyn-3-yloxy)-1,2,5-thiadiazol-4-yl)-1-azabicyclo[3.2.1]octane,
Exo (5R,6R)-6-(3-(1-(4-Chlorophenyl)-4-methyl-1-pentyn-3-yloxy)-1 ,2,5-thiadiazol-4-yl)-1-azabicyclo[3.2.1]octane,
Exo (5R,6R)-6-(3-(3-(2-Thienyl)-1-methyl-2-propyn-1-yloxy)-1,2,5-thiadiazol-4-yl)-1-azabicyclo[3.2.1]octane,
Exo (5R,6R)-6-(3-(3-(3-Thienyl)-1-methyl-2-propyn-1-yloxy)-1,2,5-thiadiazol-4-yl)-1-azabicyclo[3.2.1]octane,
Exo (5R,6R)-6-(3-[3-(2-Thienyl)-2-propyn-1-yloxy]-1,2,5-thiadiazol-4-yl)-1-azabicyclo[3.2.1]octane,
Exo (5R,6R)-6-[3-[3-(3-Thienyl)-2-propyn-1-yloxy]-1,2,5-thiadiazol-4-yl)-1-azabicyclo[3.2.1]octane,
Exo (5R,6R)-6-(3-(1-(3-Thienyl)-4-methyl-1-pentyn-3-yloxy)-1,2,5-thiadiazol-4-yl)-1-azabicyclo[3.2.1]octane,
Exo (5S,6S)-6-(3-(3-Phenyl-2-propyn-1-yloxy)-1,2,5-thiadiazol-4-yl)-1-azabicyclo[3.2.1]octane,
(+-)3-(3-(3-Phenyt-2-propyn-1-y)oxy)-1.2,5-thiad)azo)-4-y))-1-azabicyc)o[2.2.2]octane,
(+-)3-(3-(3-(4-Fluorophenyl)-2-propyn-1-yloxy)-1,2,5-thiadiazol-4-yl)-1-azabicyclo[2.2.2]octane,
(+-) 3-(3-(3-(4-Chlorophenyl)-2-propyn-1-ylthio)-1,2,5-thiadiazol-4-yl)-1-azabicyclo[2.2.2]octane,
Endo (α)-3-(3-(3-(3,5-Difluorophenyl)-2-propyn-1-yloxy)-1,2,5-thiadiazol-4-yl)-1-azabicyclo[2.2.1]heptane,
Endo (α)-3-(3-(3-(3-Chloro-5-fluorophenyl)-2-propyn-1-yloxy)-1,2,5-thiadiazol-4-yl)-1-azabicyclo[2.2.1]heptane,
Endo (β)-3-(3-(3-(3-Furyl)-2-propyn-1-yloxy)-1,2,5-thiadiazol-4-yl)-1-azabicyclo[2.2.1]heptane,
Endo (β)-3-(3-(3-(3-Cyanophenyl)-2-propyn-1-yloxy)-1,2,5-thiadiazol-4-yl)-1-azabicyclo[2.2.1]heptane,
Endo (β)-3-(3-(3-(3-Trifluoromethoxyphenyl)-2-propyn-1-yloxy)-1,2,5-thiadiazol-4-yl)-1-azabicyclo[2.2.1]heptane,
Endo (β)-3-(3-(3-(3-Chlorophenyl)-2-propyn-1-yloxy)-1,2,5-thiadiazol-4-yl)-1-azabicyclo[2.2.1]heptane,
Endo (β)-3-(3-(3-(3,5-Dichlorophenyl)-2-propyn-1-yloxy)-1,2,5-thiadiazol-4-yl)-1-azabicyclo[2.2.1]heptane,
Endo (β)-3-(3-(3-(3,5-Difluorophenyl)-2-propyn-1-yloxy)-1,2,5-thiadiazol-4-yl)-1-azabicyclo[2.2.1]heptane,
Endo (β)-3-(3-(3-(3-Bromophenyl)-2-propyn-1-yloxy)-1,2,5-thiadiazol-4-yl)-1-azabicyclo[2.2.1 ]heptane,
Endo (β)-3-(3-(3-(3-Fluorophenyl)-2-propyn-1-yloxy)-1,2,5-thiadiazol-4-yl)-1-azabicyclo[2.2.1]heptane,
Endo (β)-3-(3-(3-(3-Chloro-5-fluorophenyl)-2-propyn-1-yloxy)-1,2,5-thiadiazol-4-yl)-1-azabicyclo[2.2.1]heptane,
Endo (β)-3-(3-(3-(3-Bromo-5-fluorophenyl)-2-propyn-1-yloxy)-1,2,5-thiadiazol-4-yl)-1-azabicyclo[2.2.1]heptane,
Endo (β)-3-(3-(3-(5-Chloro-2-thienyl)-2-propyn-1-yloxy)-1,2,5-thiadiazol-4-yl)-1-azabicyclo[2.2.1]heptane,
Endo (α)-3-(3-(3-(4-Chlorophenyl)-2-propyn-1-ylthio)-1,2,5-thiadiazol-4-yl)-1-azabicyclo[2.2.1]heptane,
Endo (α)-3-(3-(3-(3,5-Difluorophenyl)-2-propyn-1-ylthio)-1,2,5-thiadiazol-4-yl)-1-azabicyclo[2.2.1]heptane,
Endo (α)-3-(3-(3-(3-Chloro-5-fluorophenyl)-2-propyn-1-ylthio)-1,2,5-thiadiazol-4-yl)-1-azabicyclo[2.2.1]heptane,
(3S) 3-(3-(1-(4-Chlorophenyl)-4-methyl-1-pentyn-3-yloxy)-1,2,5-thiadiazol-4-yl)-1-azabicyclo[2.2.2]octane,
(3S) 3-(3-(1-(3-Chlorophenyl)-4-methyl-1-pentyn-3-yloxy)-1,2,5-thiadiazol-4-yl)-1-azabicyclo[2.2.2]octane,
(3S) 3-(3-(1-(4-Fluorophenyl)-4-methyl-1-pentyn-3-yloxy)-1,2,5-thiadiazol-4-yl)-1-azabicyclo[2.2.2]octane,
(3S) 3-(3-(3-(3,5-Dichlorophenyl)-2-propyn-1-yloxy)-1,2,5-thiadiazol-4-yl)-1-azabicyclo[2.2.2]octane,
(3S) 3-(3-(3-(3-Bromophenyl)-2-propyn-1-yloxy)-1,2,5-thiadiazol-4-yl)-1-azabicyclo[2.2.2]octane,
(3S) 3-(3-(3-(3-Trifluoromethoxyphenyl)-2-propyn-1-yloxy)-1,2,5-thiadiazol-4-yl )-1-azabicyclo[2.2.2]octane,
(3S) 3-{3-(3-(3,5-Difluorophenyl)-2-propyn-1-yloxy)-1 ,2,5-thiadiazol-4-yl)-1-azabicyclo[2.2.2]octane,
(3S) 3-(3-(3-(3-Chloro-5-fIuorophenyl)-2-propyn-1-yloxy)-1.2,5-thiadiazol-4-yl)-1-azabicyclo[2.2.2]octane,
(3S) 3-(3-(3-(3-Bromo-5-fluorophenyl)-2-propyn-1-yloxy)-1 ,2,5-thiadiazol-4-yl)-1-azabicyclo[2.2.2]octane,
(3S) 3-(3-(3-(5-Chloro-2-thienyl)-2-propyn-1-yloxy)-1,2,5-thiadiazol-4-yl)-1-azabicyclo[2.2.2]octane,
Endo (5R,6S)-6-(3-(3-(2-Fluorophenyl)-2-propyn-1-ylthio)-1,2,5-thiadiazol-4-yl)-1-azabicyclo[3.2.1]octane,
Endo (5R,6S)-6-(3-(3-(3-Methylthiophenyl)-2-propyn-1-ylthio)-1,2,5-thiadiazol-4-yl)-1-azabicyclo[3.2.1]octane,
Endo (5R,6S)-6-(3-(1-(3-Methoxyphenyl)-4-methyl-1-pentyn-3-ylthio)-1,2,5-thiadiazol-4-yl)-1-azabicyclo[3.2.1]octane,
Endo (5R,6S)-6-(3-(1-(3-Methoxyphenyl)l-1-pentyn-3-ylthio)-1,2,5-thiadiazol-4-yl)-1-azabicyclo[3.2.1]octane,
Endo (5R,6S)-6-(3-(I-(2-Thienyl)-4-methyl-l-pentyn-3-ylthio)-1,2,5-thiadiazol-4-yi)-1-azabicyclo[3.2.1]octane,
Endo (5R,6S)-6-(3-(3-(3,5-Difluorophenyl)-2-propyn-1-ylthio)-1,2,5-thiadiazol-4-yl)-1-azabicyclo[3.2.1]octane,
Exo (5R,6R)-6-(3-(1-(3-Chlorophenyl)-4-methyl-1-pentyn-3-ylthio)-1,2,5-thiadiazol-4-yl)-1-azabicyclo[3.2.1]octane,
Exo (5R,6R)-6-(3-(1-(3,5-Difluorophenyl)-4-methyl-1-pentyn-3-ylthio)-1,2,5-thiadiazol-4-yl)-1-azabicyclo[3.2.1]octane,
Exo (5R,6R)-6-(3-(1-(3-Cyanophenyl)-4-methyl-1-pentyn-3-ylthio)-1,2,5-thiadiazol-4-yl)-1-azabicyclo[3.2.1]octane,
Exo (5R,6R)-6-(3-(3-(3-Bromophenyl)-2-propyn-1-ylthio)-1,2,5-thiadiazol-4-yl)-1-azabicyclo[3.2.1]octane,
Exo (5R,6R)-6-(3-(3-(3-Chloro-5-fluorophenyl)-2-propyn-1-ylthio)-1,2,5-thiadiazol-4-yl)-1-azabicyclo[3.2.1]octane.
Exo (5R,6R)-6-(3-(3-(3-Bromo-5-fluorophenyl)-2-propyn-1-ylthio)-1,2,5-thiadiazol-4-yl)-1-azabicyclo[3.2.1]octane,
Exo (5R,6R)-6-(3-(3-(5-Pyrimidyl)-2-propyn-1-ylthio)-1,2,5-thiadiazol-4-yl)-1-azabicyclo[3.2.1]octane,
Exo (5R,6R)-6-(3-(4-methyl-1-phenyl-1-pentyn-3-yloxy)-1,2,5-thiadiazol-4-yl)-1-azabicyclo[3.2.1]octane,
Exo (5R,6R)-6-(3-(1-(3,5-Dichlorophenyt)-4-methyl-1-pentyn-3-yloxy)-1,2,5-thiadiazot-4-yl)-1-azabicyclo[3.2.1]octane,
Exo (5R,6R)-6-(3-(3-(3-Cyanophenyl)-2-propyn-1-yloxy)-1,2,5-thiadiazol-4-yl)-1-azabicyclo[3.2.1]octane,
Exo (5R,6R)-6-(3-(1-(3-Chlorophenyl)-4-methyl-1-pentyn-3-yloxy)-1,2,5-thiadiazol-4-yl)-1-azabicyclo[3.2.1]octane,
Exo (5R,6R)-6-(3-(1-(3-Cyanophenyl)-4-methyl-1-pentyn-3-yloxy)-1,2,5-thiadiazol-4-yl)-1-azabicyclo[3.2.1]octane,
Exo (5R,6R)-6-(3-(1-(3,5-Difluorophenyl)-4-methyl-1-pentyn-3-yloxy)-1,2,5-thiadiazol-4-yl)-1-azabicyclo[3.2.1]octane,
Exo (5R,6R)-6-(3-(3-(3,5-Difluorophenyl)-2-propyn-1-yloxy)-1,2,5-thiadiazol-4-yl)-1-azabicyclo[3.2.1]octane,
Exo (5R,6R)-6-(3-(3-(3-Bromophenyl)-2-propyn-1-yloxy)-1,2,5-thiadiazol-4-yl)-1-azabicyclo[3.2.1]octane,
Exo (5R,6R)-6-(3-(3-(3-Chtoro-5-fluorophenyl)-2-propyn-1-yloxyr1,2,5-thiadiazot-4-yl)-1-azabicyclo[3.2.1]octane,
Exo (5R,6R)-6-(3-(3-(3-Bromo-5-fluarophenyl)-2-propyn-1-yloxy)-1,2,5-thiadiazol-4-yl)-1-azabicyclo[3.2.1]octane,
Exo (5R,6R)-6-(3-(1-(2-Thienyl)-4-methyl-1-pentyn-3-yloxy)-1,2,5-thiadiazol-4-yl)-1-azabicyclo[3.2.1]octane,
Exo (5R,6R)-6-(3-(3-(3-Furyl)-2-propyn-1-yloxy)-1,2,5-thiadiazol-4-yl)-1-azabicyclo[3.2.1]octane,
Endo (β) 3-(3-(3-(3,5-Difluorophenyl)-2-propyn-1-ylthio)-1,2,5-thiadiazol-4-yl)-1-azabicyclo[2.2.1]heptane,
Endo (β) 3-(3-(3-(3-Fluorophenyl)-2-propyn-1-ylthio)-1,2,5-thiadiazol-4-yl)-1-azabicyclo[2.2.1]heptane,
Endo (β) 3-(3-(3-(3-Chloro-5-fluorophenyl)-2-propyn-1-ylthio)-1,2,5-thiadiazol-4-yl)-1-azabicyclo[2.2.1]heptane,
Endo (β) 3-(3-(3-(3-Bromo-5-fluorophenyl)-2-propyn-1-ylthio)-1,2,5-thiadiazol-4-yl)-1-azabicyclo[2.2.1]heptane,
(+-) 3-(3-(3-(3-Chlorophenyl)-2-propyn-1-ylthio)-1,2,5-thiadiazol-4-yl)-1-azabicyclo[2.2.2]octane,
(3S) 3-(3-(3-(3-Fluorophenyl)-2-propyn-1-ylthio)-1,2,5-thiadiazol-4-yl)-1-azabicyclo[2.2.2]octane,
(3S) 3-(3-(3-(3,5-Dichlorophenyl)-2-propyn-1-ylthio)-1,2,5-thiadiazol-4-yl)-1-azabicyclo[2.2.2]octane,
(3S) 3-(3-(3-(3-Bromophenyl)-2-propyn-1-ylthio)-1,2,5-thiadiazol-4-yl)-1-azabicyclo[2.2.2]octane,
(3S) 3-(3-(3-(3-Trifluoromethoxyphenyl)-2-propyn-1-ylthio)-1,2,5-thiadiazol-4-yl)-1-azabicyclo[2.2.2]octane,
(3S) 3-(3-(1-(3,5-Difluorophenyl)-4-methyl-1-pentyn-3-ylthio)-1,2,5-thiadiazol-4-yl)-1-azabicyclo[2.2.2]octane,
(3S) 3-(3-(3-(3,5-Difluorophenyl)-2-propyn-1-ylthio)-1,2,5-thiadiazol-4-yl)-1-azabicyclo[2.2.2]octane,
(3S) 3-(3-(3-(3-Bromo-5-fluorophenyl)-2-propyn-1-ylthio)-1,2,5-thiadiazol-4-yl)-i-azabicyclo[2.2.2]octane,
(3S) 3-(3-(3-(5-Chloro-2-thienyl)-2-propyn-1-ylthio)-1,2,5-thiadiazol-4-yl)-1-azabicyclo[2.2.2]octane,
or a pharmaceutically acceptable salt or solvate thereof.

14. A method of preparing a compound according to claim 1, **characterized in**,
a) reaction of iodine or bromine substituted aromates and propargylalcohols in the presence of a palladium catalyst 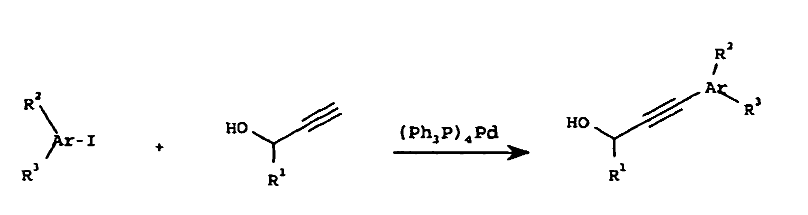 wherein Ar, R¹, R² and R³ have the meanings defined above which alcohol can be reacted with an azabicyclic 1,2,5-thiadiazole under basic conditions 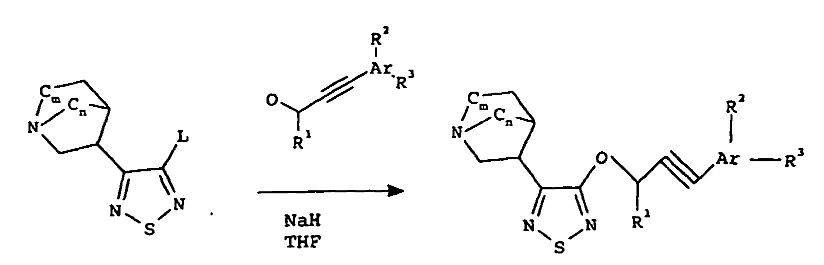 wherein L is a leaving group e.g chlorine or SO₂R, wherein R is alkyl or phenyl and Ar, R¹, R², R³, n and m have the meanings defined above; or
b) compounds wherein X is S can be synthesized by displacing the leaving group L with sulfur e.g. using NaSH, and then alkylating on the sulfur atom with P-propargylalcohol, wherein O-P is a leaving group e.g O-SO₂R or O-"Mitsunobu", wherein R has the meaning defined above, 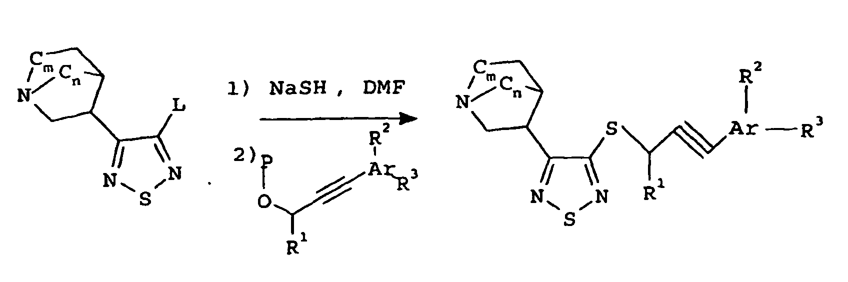 and wherein Ar, R¹, R², R³, n and m have the meanings defined above.

15. A pharmaceutical composition comprising a compound according to claim 1 together with one or more pharmaceutically acceptable carriers or diluents.

16. A pharmaceutical composition for use in treating a disease in the central nervous system caused by malfunctioning of the muscarinic cholinergic system comprising an effective amount of a compound according to claim 1 together with a pharmaceutically acceptable carrier or diluent.

17. The pharmaceutical composition according to claim 15 or 16 in the form of an oral dosage unit or parenteral dosage unit.

18. The pharmaceutical composition according to claim 17, wherein said dosage unit comprises from about 0.1 to about 100 mg of the compound according to claim 1.

19. The use of a compound according to claim 1 or a pharmaceutically acceptable salt thereof for the preparation of a medicament for treatment of a disease in the central nervous system caused by malfunctioning of the muscarinic cholinergic system.

20. The use of a compound according to claim 1 or a pharmaceutically acceptable salt thereof for the preparation of a medicament for treatment of a condition associated with the modulation of a muscarinic cholinergic receptor.

21. The use of a compound according to claim 1 or a pharmaceutically acceptable salt hereof for the preparation of a medicament for interacting with a muscarinic cholinergic receptor.

## Patentansprüche

1. Verbindung der Formel I 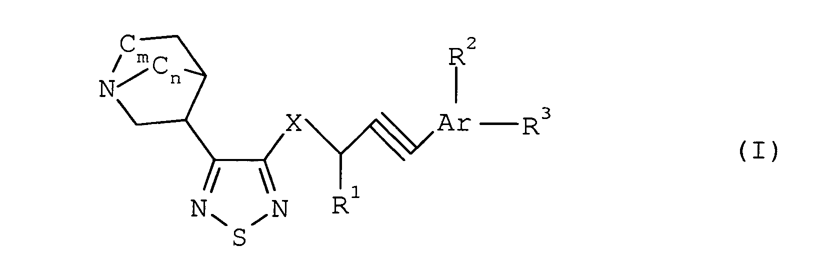 wobei
X Sauerstoff oder Schwefel ist und
R¹ Wasserstoff, geradkettiges oder verzweigtes C₁₋₅-Alkyl, geradkettiges oder verzweigtes C₂₋₅-Alkenyl, geradkettiges oder verzweigtes C₂₋₅-Alkinyl oder geradkettiges oder verzweigtes C₄₋₅-Alkeninyl ist, wobei jedes davon wahlweise mit einem oder mehreren Halogen(en) substituiert ist und
R² and R³ unabhängig voneinander Wasserstoff, Halogen, CN, NO₂, CF₃, OCF₃, C₁₋₃-Alkyl, C₁₋₃-Alkoxy oder C₁₋₃-Alkylthio sind, wobei C₁₋₃-Alkyl, C₁₋₃-Alkoxy und C₁₋₃-Alkylthio wahlweise mit einem oder mehreren Halogen(en), Cyano, Amino oder Nitro substituiert sind, und
Ar Phenyl, Thienyl, Pyridyl, Pyrimidinyl, Thiazolyl oder Furyl ist und
n 0, 1 oder 2 ist und
m 0, 1 oder 2 ist, oder
ein pharmazeutisch vertraghches Salz oder Solvat davon.

2. Verbindung der Formel I 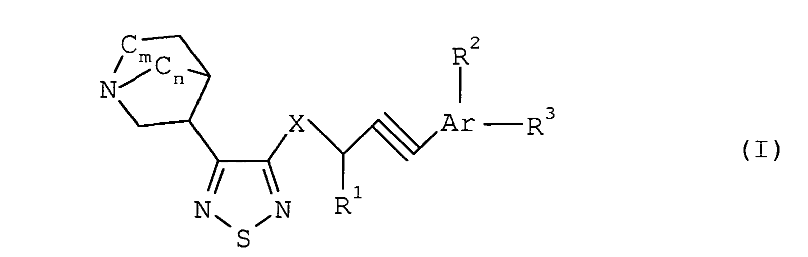 wobei
X Sauerstoff oder Schwefel ist und
R¹ Wasserstoff, geradkettiges oder verzweigtes C₁₋₅-Alkyl, geradkettiges oder verzweigtes C₂₋₅-Alkenyl, geradkettiges oder verzweigtes C₂₋₅-Allcinyl oder geradkettiges oder verzweigtes C₄₋₅-Alkeninyl, wobei jedes davon wahlweise mit einem oder mehreren Halogen(en) substituiert ist, und
R² und R³ unabhängig voneinander Wasserstoff, Halogen, CN, NO₂, C₁₋₃-Alkyl, C₁₋₃-Akoxy oder C₁₋₃-Akylthio sind, wobei C₁₋₃-Akyl, C₁₋₃-Alkoxy und C₁₋₃-Alkylthio wobei jedes davon wahlweise mit einem der mehreren Halogen(en), Cyano, Amino oder Nitro substituiert ist, und
Ar Phenyl, Thienyl, Pyridyl, Pyrimidinyl, Thiazolyl oder Furyl ist und
n 0, 1 oder 2 ist und
m 0, 1 oder 2 ist, oder
ein pharmazeutisch verträgliches Salz oder Solvat davon.

3. Verbindung nach Anspruch 1 oder 2, wobei X Sauerstoff ist.

4. Verbindung nach Anspruch 1 oder 2, wobei X Schwefel ist.

5. Verbindung nach den Ansprüchen 1 bis 4, wobei R¹ Wasserstoff ist.

6. Verbindung nach den Ansprüchen 1 bis 4, wobei R¹ geradkettiges oder verzweigtes C₁₋₅-Alkyl ist.

7. Verbindung nach den Ansprüchen 1 bis 4 und 6, wobei R¹ geradkettiges oder verzweigtes C₁₋₃-Alkyl ist.

8. Verbindung nach den Ansprüchen 1 bis 4 und 6 und 7, wobei R¹ Isopropyl ist.

9. Verbindung nach einem der vorangehenden Ansprüche, wobei R² und R³ Halogen(e) sind.

10. Verbindung nach einem der vorangehenden Ansprüche, wobei Ar Phenyl oder Thienyl ist.

11. Verbindung nach einem der vorangehenden Ansprüche, wobei m 1 oder 2 ist.

12. Verbindung nach einem der vorangehenden Ansprüche, wobei n 1 oder 2 ist.

13. Verbindung nach Anspruch 1, die ausgewählt ist aus Folgendem:
Endo-(+-)-3-(3-(3-Phenyl-2-propin-1-yloxy)-1,2,5-thiadiazol-4-yl)-1-azabicyclo[2.2.1]heptan,
Endo-(+-)-3-(3-(3-(4-Fluorphenyl)-2-propin-1-yloxy)-1,2,5-thiadiazol-4-yl)-1-azabicyclo[2.2.1]heptan,
Endo-(α)-3-(3-(3-(4-Fluorphenyl)-2-propin-1-yloxy)-1,2,5-thiadiazol-4-yl)-1-azabicyclo[2.2.1]heptan,
Endo-(α)-3-(3-(3-Phenyl-2-propin-1-yloxy)-1,2,5-thiadiazol-4-yl)-1-azabicyclo[2.2.1]heptan,
Endo-(α)-3-(3-(3-(3-Methoxyphenyl)-2-propin-1-yloxy)-1,2,5-thiadiazol-4-yl)-1-azabicyclo[2.2.1]heptan,
Endo-(α)-3-(3-(3-(4-Chlorphenyl)-2-propin-1-yloxy)-1,2,5-thiadiazol-4-yl)-1-azabicyclo[2.2.1]heptan,
Endo-(α)-3-(3-[3-(3-Thienyl)-2-propin-1-yloxy]-1,2,5-thiadiazol-4-yl)-1-azabicyclo[2.2.1]heptan,
Endo-(α)-3-(3-(3-(2-Thienyl)-2-propin-1-yloxy)-1,2,5-thiadiazol-4-yl)-1-azabicyclo[2.2.1]heptan,
Endo-(β)-3-(3-(3-(4-Fluorphenyl)-2-propin-1-yloxy)-1,2,5-thiadiazol-4-yl)-1-azabicyclo[2.2.1]heptan,
Endo-(β)-3-(3-(3-(3-Methoxyphenyl)-2-propin-1-yloxy)-1,2,5-thiadiazol-4-yl)-1-azabicyclo[2.2.1]heptan,
Endo-(β)-3-(3-(3-Phenyl-2-propin-1-yloxy)-1,2,5-thiadiazol-4-yl)-1-azabicyclo[2.2.1]heptan,
Endo-(α)-3-(3-(3-Phenyl-2-propin-1-ylthio)-1,2,5-thiadiazol-4-yl)-1-azabicyclo[2.2.1]heptan,
Exo-(+-)-3-(3-(3-Phenyl-2-propin-1-yloxy)-1,2,5-thiadiazol-4-yl)-1-azabicyclo[2.2.1]heptan,
Exo-(+-)-3-(3-(1-(3-Methoxyphenyl)-4-methyl-1-pentin-3-yloxy)-1,2,5-thiadiazol-4-yl)-1-azabicyclo[2.2.1]heptan,
Exo-(+-)-3-(3-(1-(4-Chlorphenyl)-4-methyl-1-pentin-3-yloxy)-1,2,5-thiadiazol-4-yl)-1-azabicyclo[2.2.1]heptan,
Exo-(+-)-3-(3-(1-(3-Thienyl)-4-methyl-1-pentm-3-yloxy)-1,2,5-thiadiazol-4-yl)-1-azabicyclo[2.2.1]heptan,
Exo-(+-)-3-(3-(3-(4-Chlorphenyl)-2-propm-1-ylthio)-1,2,5-thiadiazol-4-yl)-1-azabicyclo[2.2.1]heptan,
(3S)-3-(3-(3-(4-Chlorphenyl)-2-propin-1-yloxy)-1,2,5-thiadiazol-4-yl)-1-azabicyclo[2.2.2]octan,
(3S)-3-(3-(3-(3-Chlorphenyl)-2-propin-1-yloxy)-1,2,5-thiadiazol-4-yl)-1-azabicyclo[2.2.2]octan,
Endo-(5R,6S)-6-(3-(3-Phenyl-2-propin-1-ylthio)-1,2,5-thiadiazol-4-yl)-1-azabicyclo[3.2.1]octan,
Endo-(5R,6S)-6-(3-(3-(3-Fluorphenyl)-2-propin-1-ylthio)-1,2,5-thiadiazol-4-yl)-1-azabicyclo[3.2.1]octan,
Endo-(5R,6S)-6-(3-(3-(4-Fluorphenyl)-2-propin-1-ylthio)-1,2,5-thiadiazol-4-yl)-1-azabicyclo[3.2.1]octan,
Endo-(5R,6S)-6-(3-(3-(4-Chlorphenyl)-2-propin-1-ylthio)-1,2,5-thiadiazol-4-yl)-1-azabicyclo[3.2.1]octan,
Endo-(5R,6S)-6-(3-(3-(3-Thienyl)-2-propin-1-ylthio)-1,2,5-thiadiazol-4-yl)-1-azabicyclo[3.2.1]octan,
Endo-(5R,6S)-6-(3-(3-(2-Thienyl)-2-propin-1-ylthio)-1,2,5-thiadiazol-4-yl)-1-azabicyclo[3.2.1]octan,
Endo-(5R,6S)-6-(3-(3-Phenyl-2-propin-1-yloxy)-1,2,5-thiadiazol-4-yl)-1-azabicyclo [3.2..1]octan,
Endo-(SR,6S)-6-(3-(3-(4-Fluorphenyl)-2-propin-1-yloxy)-1,2,5-thiadiazol-4-yl)-1-azabicyclo[3.2.1]octan,
Endo-(5R,6S)-6-(3-(3-(3-Methoxyphenyl)-2-propin-1-yloxy)-1,2,5-thiadiazol-4-yl)-1-azabicyclo[3.2.1]octan,
Endo-(5R,6S)-6-(3-(3-(4-Chlorphenyl)-2-propin-1-yloxy)-1,2,5-thiadiazol-4-yl)-1-azabicyclo[3.2.1]octan,
Endo-(5R,6S)-6-(3-[3-(3-Thienyl)-2-propin-1-yloxy]-1,2,5-thiadiazol-4-yl)-1-azabicyclo[3.2.1]octan,
Endo-(5R,6S)-6-(3-[3-(3-Pyridyl)-2-propin-1-yloxy]-1,2,5-thiadiazol-4-yl)-1-azabicyclo[3.2.1]octan,
Endo-(5S,6R)-6-(3-(3-Phenyl-2-propin-1-yloxy)-1,2,5-thiadiazol-4-yl)-1-azabicyclo[3.2.1]octan,
Endo-(5S,6R)-6-(3-(3-(4-Fluorphenyl)-2-propin-1-yloxy)-1,2,5-thiadiazol-4-yl)-1-azabicyclo[3.2.1]octan,
Endo-(5S,6R)-6-(3-[3-(3-Methylthiophenyl)-2-propin-1-yloxy]-1,2,5-thiadiazol-4-yl)-1-azabicyclo[3.2.1]octan,
Endo-(5S,6R)-6-(3-[1-(3-Methoxyphenyl)-4-methyl-1-pentin-3-yloxy]-1,2,5-thiadiazol-4-yl)-1-azabicyclo[3.2.1]octan,
Endo-(5S,6R)-6-(3-[3-(2,5-Difluorphenyl)-2-propin-1-yloxy]-1,2,5-thiadiazol-4-yl)-1-azabicyclo[3.2.1]octan,
Endo-(5S,6R)-6-(3-[3-(3,5-Difluorphenyl)-2-propin-1-yloxy]-1,2,5-thiadiazol-4-yl)-1-azabicyclo[3.2.1]octan,
Endo-(5S,6R)-6-(3-[1-(2-Thienyl)-4-methyl-1-pentin-3-yloxy]-1,2,5-thiadiazol-4-yl)-1-azabicyclo[3.2.1]octan,
Exo-(5R,6R)-6-(3-(3-Phenyl-2-propin-1-ylthio)-1,2,5-thiadiazol-4-yl)-1-azabicyclo[3.2.1]octan,
Exo-(5R,6R)-6-(3-(3-Phenyl-2-propin-1-yloxy)-1,2,5-thiadiazol-4-yl)-1-azabicyclo[3.2.1]octan,
Exo-(5R,6R)-6-(3-(3-(3-Methoxyphenyl)-2-propin-1-yloxy)-1,2,5-thiadiazol-4-yl)-1-azabicyclo[3.2.1]octan,
Exo-(5R,6R)-6-(3-(3-(3-Trifluormethylphenyl)-2-propin-1-yloxy)-1,2,5-thiadiazol-4-yl)-1-azabicyclo[3.2.1]octan,
Exo-(5R,6R)-6-(3-(3-(3-Methoxyphenyl)-1-methyl-2-propin-1-yloxy)-1,2,5-thiadiazol-4-yl)-1-azabicyclo[3.2.1]octan,
Exo-(5R,6R)-6-(3-(3-(4-Chlorphenyl)-2-propin-1-yloxy)-1,2,5-thiadiazol-4-yl)-1-azabicyclo[3.2.1]octan,
Exo-(5R,6R)-6-(3-(1-(3-Methoxyphenyl)-4-methyl-1-pentin-3-yloxy)-1,2,5-thiadiazol-4-yl)-1-azabicyclo[3.2.1]octan,
Exo-(5R,6R)-6-(3-(1-(4-Chlorphenyl)-4-methyl-1-pentin-3-yloxy)-1,2,5-thiadiazol-4-yl)-1-azabicyclo[3.2.1]octan,
Exo-(5R,6R)-6-(3-(3-(2-Thienyl)-1-methyl-2-propin-1-yloxy)-1,2,5-thiadiazol-4-yl)-1-azabicyclo[3.2.1]octan,
Exo-(5R,6R)-6-(3-(3-(3-Thienyl)-1-methyl-2-propin-1-yloxy)-1,2,5-thiadiazol-4-yl)-1-azabicyclo[3.2.1]octan,
Exo-(5R,6R)-6-(3-[3-(2-Thienyl)-2-propin-1-yloxy]-1,2,5-thiadiazol-4-yl)-1-azabicyclo[3.2.1]octan,
Exo-(SR,6R)-6-(3-[3-(3-Thienyl)-2-propin-1-yloxy]-1,2,5-thiadiazol-4-yl)-1-azabicyclo[3.2.1]octan,
Exo-(5R,6R)-6-(3-(1-(3-Thienyl)-4-methyl-1-pentin-3-yloxy)-1,2,5-thiadiazol-4-yl)-1-azabicyclo[3.2.1]octan,
Exo-(5S,6S)-6-(3-(3-Phenyl-2-propin-1-yloxy)-1,2,5-thiadiazol-4-yl)-1-azabicyclo[3.2.1]octan,
(+-)-3-(3-(3-Phenyl-2-propin-1-yloxy)-1,2,5-thiadiazol-4-yl)-1-azabicyclo [2.2. 2] octan,
(+-)-3-(3-(3-(4-Fluorphenyl)-2-propin-1-yloxy)-1,2,5-thiadiazol-4-yl)-1-azabicyclo[2.2.2]octan,
(+-)-3-(3-(3-(4-Chlorphenyl)-2-propin-1-ylthio)-1,2,5-thiadiazol-4-yl)-1-azabicyclo[2.2.2]octan,
Endo-(α)-3-(3-(3-(3,5-Difluorphenyl)-2-propin-1-yloxy)-1,2,5-thiadiazol-4-yl)-1-azabicyclo[2.2.1]heptan,
Endo-(α)-3-(3-(3-(3-Chlor-5-fluorphenyl)-2-propin-1-yloxy)-1,2,5-thiadiazol-4-yl)-1-azabicyclo[2.2.1]heptan,
Endo-(β)-3-(3-(3-(3-Furyl)-2-propin-1-yloxy)-1,2,5-thiadiazol-4-yl)-1-azabicyclo[2.2.1]heptan,
Endo-(β)-3-(3-(3-(3-Cyanophenyl)-2-propin-1-yloxy)-1,2,5-thiadiazol-4-yl)-1-azabicyclo[2.2.1]heptan,
Endo-(β)-3-(3-(3-(3-Tnfluormethoxyphenyl)-2-propin- 1 -yloxy)- 1,2,5-thiadiazol-4-yl)-1-azabicyclo[2.2.1]heptan,
Endo-(β)-3-(3-(3-(3-Chlorphenyl)-2-propin-1-yloxy)-1,2,5-thiadiazol-4-yl)-1-azabicyclo[2.2.1]heptan,
Endo-(β)-3-(3-(3-(3,5-Dichlorphenyl)-2-propin-1-yloxy)-1,2,5-thiadiazol-4-yl)-1-azabicyclo[2.2.1]heptan,
Endo-(β)-3-(3-(3-(3,5-Difluorphenyl)-2-propin-1-yloxy)-1,2,5-thiadiazol-4-yl)-1-azabicyclo[2.2.1]heptan,
Endo-(β)-3-(3-(3-(3-Bromphenyl)-2-propm-1-yloxy)-1,2,5-thiadiazol-4-yl)-1-azabicyclo[2.2.1]heptan,
Endo-(β)-3-(3-(3-(3-Fluorphenyl)-2-propin-1-yloxy)-1,2,5-thiadiazol-4-yl)-1-azabicyclo[2.2.1]heptan,
Endo-(β)-3-(3-(3-(3-Chlor-5-fluorphenyl)-2-propin-1-yloxy)-1,2,5-thiadiazol-4-yl)-1-azabicyclo[2.2.1]heptan,
Endo-(β)-3-(3-(3-(3-Brom-5-fluorphenyl)-2-propin-1-yloxy)-1,2,5-thiadiazol-4-yl)-1-azabicyclo[2.2.1]heptan,
Endo-(β)-3-(3-(3-(5-Chlor-2-thienyl)-2-propin-1-yloxy)-1,2,5-thiadiazol-4-yl)-1-azabicyclo[2.2.1]heptan,
Endo-(α)-3-(3-(3-(4-Chlorphenyl)-2-propm-1-ylthio)-1,2,5-thiadiazol-4-yl)-1-azabicyclo[2.2.1]heptan,
Endo-(α)-3-(3-(3-(3,5-Difluorphenyl)-2-propin-1-ylthio)-1,2,5-thiadiazol-4-yl)-1-azabicyclo[2.2.1]heptan,
Endo-(α)-3-(3-(3-(3-Chlor-5-fluorphenyl)-2-propin-1-ylthio)-1,2,5-thiadiazol-4-yl)-1-azabicyclo[2.2.1]heptan,
(3S)-3-(3-(1-(4-Chlorphenyl)-4-methyl-1-pentm-3-yloxy)-1,2,5-thiadiazol-4-yl)-1-azabicyclo[2.2.2]octan,
(3S)-3-(3-(1-(3-Chlorphenyl)-4-methyl-1-pentin-3-yloxy)-1,2,5-thiadiazol-4-yl)-1-azabicyclo[2.2.2]octan,
(3S)-3-(3-(1-(4-Fluorphenyl)-4-methyl-1-pentin-3-yloxy)-1,2,5-thiadiazol-4-yl)-1-azabicyclo[2.2.2]octan,
(3S)-3-(3-(3-(3,5-Dichlorphenyl)-2-propin-1-yloxy)-1,2,5-thiadiazol-4-yl)-1-azabicyclo[2.2.2]octan,
(3S)-3-(3-(3-(3-Bromphenyl)-2-propin-1-yloxy)-1,2,5-thiadiazol-4-yl)-1-azabicyclo [2.2.2] octan,
(3S)-3-(3-(3-(3-Trifluormethoxyphenyl)-2-propin-1-yloxy)-1,2,5-thiadiazol-4-yl)-1-azabicyclo[2.2.2]octan,
(3S)-3-(3-(3-(3,5-Difluorphenyl)-2-propm-1-yloxy)-1,2,5-thiadiazol-4-yl)-1-azabicyclo[2.2.2]octan,
(3S)-3-(3-(3-(3-Chlor-5-fluorphenyl)-2-propin-1-yloxy)-1,2,5-thiadiazol-4-yl)-1-azabicyclo[2.2.2]octan,
(3S)-3-(3-(3-(3-Brom-5-fluorphenyl)-2-propin-1-yloxy)-1,2,5-thiadiazol-4-yl)-1-azabicyclo[2.2.2]octan,
(3S)-3-(3-(3-(5-Chlor-2-thienyl)-2-propin-1-yloxy)-1,2,5-thiadiazol-4-yl)-1-azabicyclo[2.2.2]octan,
Endo-(5R,6S)-6-(3-(3-(2-Fluorphenyl)-2-propin-1-ylthio)-1,2,5-thiadiazol-4-yl)-1-azabicyclo[3.2.1]octan,
Endo-(5R,65)-6-(3-(3-(3-Methylthiophenyl)-2-propm-1-ylthio)-1,2,5-thiadiazol-4-yl)-1-azabicyclo[3.2.1]octan,
Endo-(5R,6S)-6-(3-(1-(3-Methoxyphenyl)-4-methyl-1-pentin-3-ylthio)-1,2,5-thiadiazol-4-yl)-1-azabicyclo[3.2.1]octan,
Endo-(5R,65)-6-(3-(1-(3-Methoxyphenyl)1-1-pentin-3-ylthio)-1,2,5-thiadiazol-4-yl)-1-azabicyclo[3.2.1]octan,
Endo-(5R,6S)-6-(3-(1-(2-Thienyl)-4-methyl-1-pentin-3-ylthio)-1,2,5-thiadiazol-4-yl)-1-azabicyclo[3.2.1]octan,
Endo-(5R,6S)-6-(3-(3-(3,5-Difluorphenyl)-2-propin-1-ylthio)-1,2,5-thiadiazol-4-yl)-1-azabicyclo[3.2.1]octan,
Exo-(5R,6R)-6-(3-(1-(3-Chlorphenyl)-4-methyl-1-pentin-3-ylthio)-1,2,5-thiadiazol-4-yl)-1-azabicyclo[3.2.1]octan,
Exo-(5R,6R)-6-(3-(1-(3,5-Difluorphenyl)-4-methyl-1-pentm-3-ylthio)-1,2,5-thiadiazol-4-yl)-1-azabicyclo[3.2.1]octan,
Exo-(5R,6R)-6-(3-(1-(3-Cyanophenyl)-4-methyl-1-pentin-3-ylthio)-1,2,5-thiadiazol-4-yl)-1-azabicyclo[3.2.1]octan,
Exo-(5R,6R)-6-(3-(3-(3-Bromphenyl)-2-propin-1-ylthio)-1,2,5-thiadiazol-4-yl)-1-azabicyclo[3.2.1]octan,
Exo-(5R,6R)-6-(3-(3-(3-Chlor-5-fluorphenyl)-2-propin-1-ylthio)-1,2,5-thiadiazol-4-yl)-1-azabicyclo[3.2.1]octan,
Exo-(5R,6R)-6-(3-(3-(3-Brom-5-fluorphenyl)-2-propin-1-ylthio)-1,2,5-thiadiazol-4-yl)-1-azabicyclo[3.2.1]octan,
Exo-(5R,6R)-6-(3-(3-(5-Pyrimidyl)-2-propin-1-ylthio)-1,2,5-thiadiazol-4-yl)-1-azabicyclo[3.2.1]octan,
Exo-(5R,6R)-6-(3-(4-Methyl-1-phenyl-1-pentin-3-yloxy)-1,2,5-thiadiazol-4-yl)-1-azabicyclo[3.2.1]octan,
Exo-(5R,6R)-6-(3-(1-(3,5-Dichlorphenyl)-4-methyl-1-pentin-3-yloxy)-1,2,5-thiadiazol-4-yl)-1-azabicyclo[3.2.1]octan,
Exo-(5R,6R)-6-(3-(3-(3-Cyanophenyl)-2-propin-1-yloxy)-1,2,5-thiadiazol-4-yl)-1-azabicyclo[3.2.1]octan,
Exo-(5R,6R)-6-(3-(1-(3-Chlorphenyl)-4-methyl-1-pentin-3-yloxy)-1,2,5-thiadiazol-4-yl)-1-azabicyclo[3.2.1]octan,
Exo-(5R,6R)-6-(3-(1-(3-Cyanophenyl)-4-methyl-1-pentm-3-yloxy)-1,2,5-thiadiazol-4-yl)-1-azabicyclo[3.2.1]octan,
Exo-(5R,6R)-6-(3-(1-(3,5-Difluorphenyl)-4-methyl-1-pentin-3-yloxy)-1,2,5-thiadiazol-4-yl)-1-azabicyclo[3.2.1]octan,
Exo-(5R,6R)-6-(3-(3-(3,5-Difluorphenyl)-2-propin-1-yloxy)-1,2,5-thiadiazol-4-yl)-1-azabicyclo[3.2.1]octan,
Exo-(5R,6R)-6-(3-(3-(3-Bromphenyl)-2-propin-1-yloxy)-1,2,5-thiadiazol-4-yl)-1-azabicyclo[3.2.1]octan,
Exo-(5R,6R)-6-(3-(3-(3-Chlor-5-fluorphenyl)-2-propin-1-yloxy)-1,2,5-thiadiazol-4-yl)-1-azabicyclo[3.2.1]octan,
Exo-(5R,6R)-6-(3-(3-(3-Brom-5-fluorphenyl)-2-propin-1-yloxy)-1,2,5-thiadiazol-4-yl)-1-azabicyclo[3.2.1]octan,
Exo-(5R,6R)-6-(3-(1-(2-Thienyl)-4-methyl-1-pentin-3-yloxy)-1,2,5-thiadiazol-4-yl)-1-azabicyclo[3.2.1]octan,
Exo-(5R,6R)-6-(3-(3-(3-Furyl)-2-propin-1-yloxy)-1,2,5-thiadiazol-4-yl)-1-azabicyclo[3.2.1]octan,
Endo-(β)-3-(3-(3-(3,5-Difluorphenyl)-2-propin-1-ylthio)-1,2,5-thiadiazol-4-yl)-1-azabicyclo[2.2.1]heptan,
Endo-(β)-3-(3-(3-(3-Fluorphenyl)-2-propin-1-ylthio)-1,2,5-thiadiazol-4-yl)-1-azabicyclo[2.2.1]heptan,
Endo-(β)-3-(3-(3-(3-Chlor-5-fluorphenyl)-2-propin-1-ylthio)-1,2,5-thiadiazol-4-yl)-1-azabicyclo[2.2.1]heptan,
Endo-(β)-3-(3-(3-(3-Brom-5-fluorphenyl)-2-propin-1-ylthio)-1,2,5-thiadiazol-4-yl)-1-azabicyclo[2.2.1]heptan,
(+-)-3-(3-(3-(3-Chlorphenyl)-2-propin-1-ylthio)-1,2,5-tluadiazol-4-yl)-1-azabicyclo[2.2.2]octan,
(3S)-3-(3-(3-(3-Fluorphenyl)-2-propin-1-ylthio)-1,2,5-thiadiazol-4-yl)-1-azabicyclo[2.2.2]octan,
(3S)-3-(3-(3-(3,5-Dichlorphenyl)-2-propin-1-ylthio)-1,2,5-thiadiazol-4-yl)-1-azabicyclo[2.2.2]octan,
(3S)-3-(3-(3-(3-Bromphenyl)-2-propm-1-ylthio)-1,2,5-thiadiazol-4-yl)-1-azabicyclo[2.2.2]octan,
(3S)-3-(3-(3-(3-Trifluormethoxyphenyl)-2-propin-1-ylthio)-1,2,5-thiadiazol-4-yl)-1-azabicyclo[2.2.2]octan,
(3S)-3-(3-(1-(3,5-Difluorphenyl)-4-methyl-1-pentin-3-ylthio)-1,2,5-thiadiazol-4-yl)-1-azabicyclo [2.2.2] octan,
(3S)-3-(3-(3-(3,5-Difluorphenyl)-2-propm-1 -ylthio)-1,2,5-thiadiazol-4-yl)-1-azabicyclo[2.2.2]octan,
(3S)-3-(3-(3-(3-Brom-5-fluorphenyl)-2-propm-1-ylthio)-1,2,5-thiadiazol-4-yl)-1-azabicyclo[2.2.2]octan,
(3S)-3-(3-(3-(5-Chlor-2-thienyl)-2-propin-1-ylthio)-1,2,5-thiadiazol-4-yl)-1-azabicyclo[2.2.2]octan,
oder ein pharmazeutisch verträgliches Salz oder Solvat davon.

14. Verfahren zur Herstellung einer Verbindung nach Anspruch 1, **dadurch gekennzeichnet, dass**
a) Iod- oder bromsubstituierte Aromate und Propargylalkohole in Gegenwart eines Palladiumkatalysators umgesetzt werden 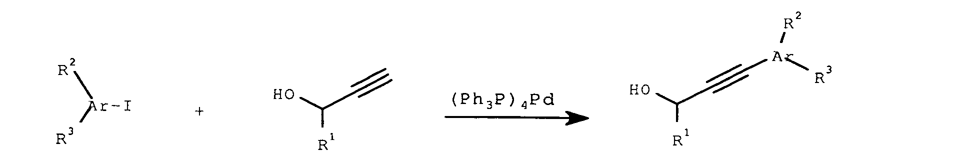 wobei Ar, R¹, R² und R³ die vorstehend definierten Bedeutungen aufweisen, wobei der Alkohol mit einem azabicyclischen 1,2,5-Thiadiazol unter basischen Bedingungen umgesetzt werden kann 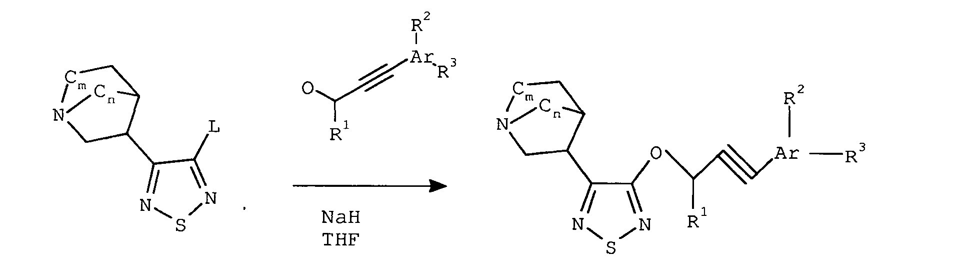 wobei L eine Abgangsgruppe, z.B. Chlor oder SO₂R ist, wobei R Alkyl oder Phenyl ist und Ar, R¹, R², R³, n und m die vorstehend definierten Bedeutungen aufweisen, oder
b) Verbindungen, in welchen X S ist, durch Ersatz der Abgangsgruppe L mit Schwefel z.B. unter Verwendung von NaSH und dann Alkylieren des Schwefelatoms mit p-Propargylalkohol synthetisiert werden können,
wobei O-P eine austretende Gruppe z.B. O-SO₂R oder O-"Mitsunobu" ist,
wobei R die vorstehend definierte Bedeutung aufweist, 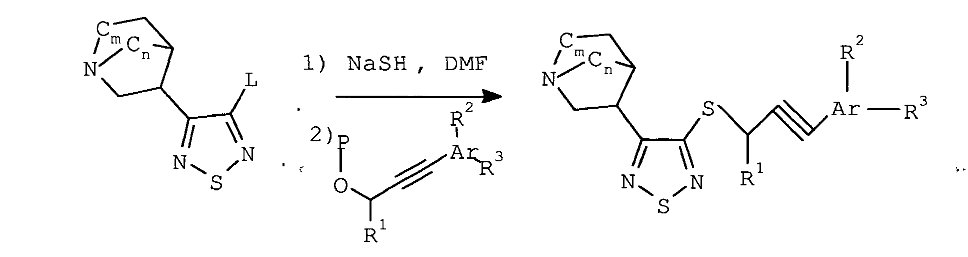 und wobei Ar, R¹, R², R³, n und m die vorstehend definierten Bedeutungen aufweisen.

15. Arzneimittel, umfassend eine Verbindung nach Anspruch 1 zusammen mit einem oder mehreren pharmazeutisch verträghchen Trägern oder Verdünnungsmitteln.

16. Arzneimittel zur Verwendung bei der Behandlung einer durch Fehlfunktion des muskarinen cholinergen Systems verursachten Erkrankung des zentralen Nervensystems, umfassend eine wirksame Menge einer Verbindung nach Anspruch 1 zusammen mit einem pharmazeutisch verträghchen Träger oder Verdünnungsmittel.

17. Arzneimittel nach Anspruch 15 oder 16 in Form einer oralen Dosierungseinheit oder parenteralen Dosierungseinheit.

18. Arzneimittel nach Anspruch 17, wobei die Dosierungseinheit etwa 0,1 bis etwa 100 mg der Verbindung nach Anspruch 1 umfasst.

19. Verwendung einer Verbindung nach Anspruch 1 oder eines pharmazeutisch verträghchen Salzes davon zur Herstellung eines Medikaments zur Behandlung einer durch Fehlfunktion des muskarinen cholinergen Systems verursachten Erkrankung des zentralen Nervensystems.

20. Verwendung einer Verbindung nach Anspruch 1 oder eines pharmazeutisch verträglichen Salzes davon zur Herstellung eines Medikaments zur Behandlung eines Zustands, der mit der Modulation eines muskarmen chohnergen Rezeptors verbunden ist.

21. Verwendung emer Verbindung nach Anspruch 1 oder eines pharmazeutisch verträglichen Salzes davon zur Herstellung emes Medikaments zum Wechselwirken mit emem muskarinen cholinergenen Rezeptor.

## Revendications

1. Un composé de formule I 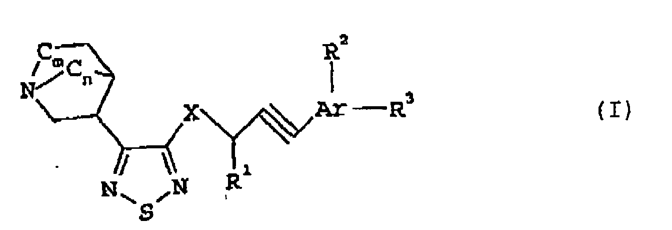 dans laquelle
X est de l'oxygène ou du soufre et
R¹ est de l'hydrogène, un radical alkyle en C₁₋₅ droit ou ramifié, un radical alcényle en C₂₋₅ droit ou ramifié, un radical alcynyle en C₂₋₅ droit ou ramifié ou un radical alcénynyle en C₄₋₅ droit ou ramifié, dont chacun est éventuellement substitué avec un ou plusieurs halogènes et
R² et R³ sont indépendamment l'hydrogéne, un halogène, CN, NO₂, CF₃, OCF₃, un radical alkyle en C₁₋₃, un radical alcoxy en C₁₋₃ ou un radical alkylthio en C₁₋₃, dans lequel le radical alkyle en C₁₋₃, le radical aldoay en C₁₋₃ et le radical alkylthio en C₁₋₃ sont éventuellement substitués avec un ou plusieurs halogènes, un radical cyano, amino ou nitro et
Ar est un radical phényle, thiényle, pyridyle, pyrimidinyle, thiazolyle ou furyle et
n est 0, 1 ou 2 et
m est 0, 1 ou 2 ou
un sel acceptable du point de vue pharmaceutique ou un produit de solvatation de celui-ci.

2. Un composé de formule (I) 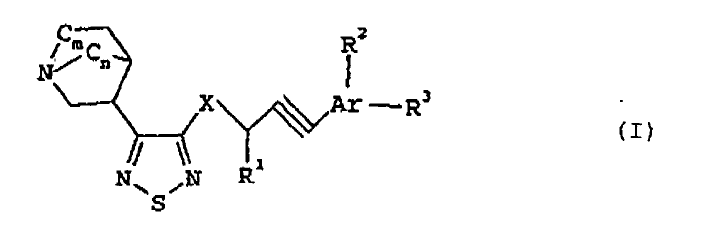 dans laquelle
X est de l'oxygène ou du soufre et
R¹ est de l'hydrogène, un radical alkyle en C₁₋₅ droit ou ramifié, un radical alcényle en C₂₋₅ droit ou ramifié, un radical alcynyle en C₂₋₅ droit ou ramifié ou un radical alcénynyle en C₄₋₅ droit ou ramifié, dont chacun est éventuellement substitué avec un ou plusieurs halogènes et
R² et R³ sont indépendamment l'hydrogène, un halogène, CN, NO₂, un radical alkyle en C₁₋₃, un radical alcoxy en C₁₋₃ ou un radical alkylthio en C₁₋₃, dans lequel le radical alkyle en C₁₋₃, le radical alcoxy en C₁₋₃ et le radical alkylthio en C₁₋₃ sont éventuellement substitués avec un ou plusieurs halogènes, un radical cyano, amino ou nitro et
Ar est un radical phényle, thiényle, pyridyle, pyrimidinyle, thiazolyle ou furyle et
n est 0, 1 ou 2 et
m est 0, 1 ou 2 ou
un sel acceptabLe du point, de vue pharmaceutique ou un produit de solvatation de celui-ci.

3. Un composé selon la revendication 1 ou 2, dans lequel X est de l'oxygène.

4. Un composé selon la revendication 1 ou 2, dans lequel X est du soufre.

5. Un composé selon les revendications 1 à 4, dans lequel R¹ est de l'hydrogène.

6. Un composé selon les revendications 1 à 4, dans lequel R¹ est un radical alkyle en C₁₋₅ droit ou ramifié.

7. Un composé selon les revendications 1 à 4 et 6, dans lequel R¹ est un radical alkyle en C₁₋₃ droit ou ramifié.

8. Un composé selon les revendications 1 à 4 et 6 et 7, dans lequel R¹ est un radical isopropyle.

9. Un composé selon l'une quelconque des revendications précédentes, dans lequel R² et R³ sont un/des halogène(s).

10. Un composé selon l'une quelconque des revendications précédentes, dans lequel Ar est un radical phényle ou thiényle.

11. Un composé selon l'une quelconque des revendications précédentes, dans lequel m est 1 ou 2.

12. Un composé selon l'une quelconque des revendications précédentes, dans lequel n est 1 ou 2.

13. Un composé selon la revendication 1, qui est choisi parmi les suivants :
l'endo(+-)3-(3-(3-phényl-2-propyn-1-yloxy)-1,2,5-thiadiazol-4-yl)-1-azabicyclo[2.2.1]heptane,
l'endo(+-)3-(3-(3-(4-fluorophényl)-2-propyn-1-yloxy)-1,2,5-thiadiazol-4-yl)-1-azabicyclo[2.2.1]heptane,
l'endo(α)3-(3-(3-(4-fluorophényl)-2-propyn-1-yloxy)-1,2,5-thiadiazol-4-yl)-1-azabicyclo[2.2.1]heptane,
l'endo(α)3-(3-(3-phényl-2-propyn-1-yloxy)-1,2,5-thiadiazol-4-yl)-1-azabicyclo[2.2.1]heptane,
l'endo(α)3-(3-(3-(3-méthoxyphényl)-2-propyn-1-yloxy)-1,2,5-thiadiazol-4-yl)-1-azabicyclo[2.2.1]heptane,
l'endo(α)3-(3-(3-(4-chlorophényl)-2-propyn-1-yloxy)-1,2,5-thiadiazol-4-yl)-1-azabicyolo[2.2.1]heptane,
l'endo(α)3-(3-[3-(3-thiényl)-2-propyn-1-yloxy]-1,2,5-thiadiazol-4-yl)-1-azabicyclo[2.2.1]heptane,
l'endo(α)3-(3-(3-(2-thiényl)-2-propyn-1-yloxy)-1,2,5-thiadiazol-4-yl)-1-azabicyclo[2.2.1]heptane,
l'endo(β)3-(3-(3-(4-fluorophényl-2-propyn-1-yloxy)-1,2,5-thiadiazol-4-yl)-1-azabicyclo[2.2.1]heptane,
l'endo(β)3-(3-(3-(3-méthoxyphényl-2-propyn-1-yloxy)-1,2,5-thiadiazol-4-yl)-1-azabicyclo[2.2.1]heptane,
l'endo(β)3-(3-(3-phényl-2-propyn-1-yloxy)-1,2,5-thiadiazol-4-yl)-1-azabicyclo[2.2.1]heptane,
l'endo(α)3-(3-(3-phényl-2-propyn-1-ylthio)-1,2,5-thiadiazol-4-yl)-1-azabzcyclo[2.2.1]heptane,
l'exo(+-)3-(3-(3-phényl-2-propyn-1-yloxy)-1,2,5-thiadiazol-4-yl)-1-azabicyclo[2.2.1]heptane,
l'exo(+-)3-(3-(1-(3-méthoxyphényl)-4-méthyl-1-pentyn-3-yloxy)-1,2,5-thiadiazol-4-yl)-1-azabicyclo[2.2.1] heptane,
l'exo(+-)3-(3-(1-(4-chlorophényl)-4-méthyl-1-pentyn-3-yloxy)-1,2,5-thiadiazol-4-yl)-1-azabicyclo[2.2.1] heptane,
l'exo(+-)3-(3-(1-(3-thiényl)-4-méthyl-1-pentyn-3-yloxy)-1,2,5-thiadiazol-4-yl)-1-azabicyclo[2.2.1]heptane,
l'exo(+-)3-(3-(3-(4-chlorophényl)-2-propyn-1-ylthio)-1,2,5-thiadiazol-4-yl)-1-azabicyclo[2.2.1]heptane,
le (3S)3-(3-(3-(4-chlorophényl)-2-propyn-1-yloxy)-1,2,5-thiadiazol-4-yl)-1-azabicyclo[2.2.2]octane,
le (3S)3-(3-(3-(3-chlorophényl)-2-propyn-1-yloxy)-1,2,5-thiadiazol-4-yl)-1-azabicyclo[2.2.2]octane,
l'endo(5R,6S)-6-(3-(3-phényl-2-propyn-1-ylthio)-1,2,5-thiadiazol-4-yl)-1-azabicyclo[3.2.1]octane,
l'endo(5R,6S)-6-(3-(3-(3-fluorophényl)-2-propyn-1-ylthio)-1,2,5-thiadiazol-4-yl)-1-azabicyclo[3.2.1]octane,
l'endo(5R,6S)-6-(3-(3-(4-fluorophényl)-2-propyn-1-ylthio)-1,2,5-thiadiazol-4-yl)-1-azabicyclo[3.2.1]octane,
l'endo(5R,6S)-6-(3-(3-(4-chlorophényl)-2-propyn-1-ylthio)-1,2,5-thiadiazol-4-yl)-1-azabicyclo[3.2.1]octane,
l'endo(5R,6S)-6-(3-(3-(3-thiényl)-2-propyn-1-ylthio)-1,2,5-thiadiazol-4-yl)-1-azabicyclo[3.2.1]octane,
l'endo(5R,6S)-6-(3-(3-(2-thiényl)-2-propyn-1-ylthio)-1,2,5-thiadiazol-4-yl)-1-azabicyclo[3.2.1]octane,
l'endo(5R,6S)-6-(3-(3-phényl-2-propyn-1-yloxy)-1,2,5-thiadiazol-4-yl)-1-azabicyclo[3.2.1]octane,
l'endo(5R,6S)-6-(3-(3-(4-fluorophényl)-2-propyn-1-yloxy)-1,2,5-thiadiazol-4-yl)-1-azabicyclo[3.2.1]octane,
l'endo(5R,6S)-6-(3-(3-(3-méthoxyphényl)-2-propyn-1-yloxy)-1,2,5-thiadiazol-4-yl)-1-azabicyclo[3.2.1]octane,
l'endo(5R,6S)-6-(3-(3-(4-chlorophényl)-2-propyn-1-yloxy)-1,2,5-thiadiazol-4-yl)-1-azabicyclo[3.2.1]octane,
l'endo(5R,6s)-6-(3-[3-(3-thiényl)-2-propyn-1-yloxy]-1,2,5-thiadiazol-4-yl)-1-azabicyclo[3.2.1]octane,
l'endo(5R,6S)-6-(3-[3-(3-pyridyl)-2-propyn-1-yloxy]-1,2,5-thiadiazol-4-yl)-1-azabicyclo[3.2.1]octane,
l'endo(5S,6R)-6-(3-(3-phényl-2-propyn-1-yloxy)-1,2,5-thiadiazol-4-yl)-1-azabicyclo[3.2.1]octane,
l'endo(5S,6R)-6-(3-(3-(4-fluorophényl)-2-propyn-1-yloxy)-1,2,5-thiadiazol-4-yl)-1-azabicyclo[3.2.1]octane,
l'endo(5S,6R)-6-(3-[3-(3-méthylthiophényl)-2-propyn-1-yloxy]-1,2,5-thiadiazol-4-yl)-1-azabicyclo[3.2.1] octane,
l'endo(5S,6R)-6-(3-[1-(3-méthoxyphényl)-4-méthyl-1-pentyn-3-yloxy]-1,2,5-thiadiazol-4-yl)-1-azabicyclo [3.2.1]octane,
l'endo(5S,6R)-6-(3-[3-(2,5-difluorophényl)-2-propyn-1-yloxy]-1,2,5-thiadiazol-4-yl)-1-azabicyclo[3.2.1] octane,
l'endo(5S,6R)-6-(3-[3-(3,5-difluorophényl)-2-propyn-1-yloxy]-1,2,5-thiadiazol-4-yl)-1-azabicyclo[3.2.1] octane,
l'endo(5S,6R)-6-(3-[1-(2-thiényl)-4-méthyl-1-pentyny-3-yloxy]-1,2,5-thiadiazol-4-yl)-1-azabicyclo[3.2.1] octane,
l'exo(5R,6R)-6-(3-(3-phényl-2-propyn-1-ylthio)-1,2,5-thiadiazol-4-yl)-1-azabicyclo[3.2.1]octane,
l'exo(5R,6R)-6-(3-(3-phényl)-2-propy:a-1-yloxy)-1,2,5-thiadiazol-4-yl)-1-azabicyClo[3.2.1]octane,
l'exo(5R,6R)-6-(3-(3-(3-méthoxyphényl)-2-propyn-1-yloxy)-1,2,5-thiadiazol-4-yl)-1-azabicyclo[3.2.1]octane,
l'exo(5R,6R)-6-(3-(3-(3-trifluorométhylphényl)-2-propyn-1-yloxy)-1,2,5-thiadiazol-4-yl)-1-azabicyclo[3.2.1] octane,
l'exo(5R,6R)-6-(3-(3-(3-méthoxyphényl)-1-méthyl-2-propyn-1-yloxy)-1,2,5-thiadiazol-4-yl)-1-azabicyclo[3.2.1] octane,
l'exo(5R,6R)-6-(3-(3-(4-chlorophényl)-2-propyn-1-yloxy)-1,2,5-thiadiazol-4-yl)-1-azabicyclo[3.2.1]octane,
l'exo(5R,6R)-6-(3-(1-(3-méthoxyphényl)-4-méthyl-1-pentyn-3-yloxy)-1,2,5-thiadiazol-4-yl)-1-azabicyclo[3.2.1] octane,
l'exo(5R,6R)-6-(3-(1-(4-chlorophényl)-4-méthyl-1-pentyn-3-yloxy)-1,2,5-thiadiazol-4-yl)-1-azabicyclo[3.2.1] octane,
l'exo(5R,6R)-6-(3-(3-(2-thiényl)-1-méthyl-2-propyn-1-yloxy)-1,2,5-thiadiazol-4-yl)-1-azabicyclo[3.2.1]octane,
l'exo(5R,6R)-6-(3-(3-(3-thiényl)-1-méthyl-2-propyn-1-yloxy)-1,2,5-thiadiazol-4-yl)-1-azabicyclo[3.2.1]octane,
l'exo(5R,6R)-6-(3-[3-(2-thiényl)-2-propyn-1-yloxy]-1,2,5-thiadiazol-4-yl)-1-azabicyclo[3.2.1]octane,
l'exo(5R,6R)-6-(3-[3-(3-thiényl)-2-propyn-1-yloxy)-1,2,5-thiadiazol-4-yl)-1-azabicyclo[3.2.1]octane,
l'exo(5R,6R)-6-(3-(1-(3-thiényl)-4-méthyl-3-pentyn-3-yloxy)-1,2,5-thiadiazol-4-yl)-1-azabicyclo[3.2.1]octane,
l'exo(5S,6S)-6-(3-(3-phényl-2-propyn-1-yloxy)-1,2,5-thiadiazol-4-yl)-1-azabicyclo[3.2.1]octane,
le (+-)3-(3-(3-phényl-2-propyn-1-yloxy)-1,2,5-thiadiazol-4-yl)-1-azabicyclo[2.2.2]octane,
le (+-)3-(3-(3(4-fluorophényl)-2-propyn-1-yloxy)-1,2,5-thiadiazol-4-yl)-1-azabicyclo[2.2.2]octane,
le (+-)3-(3-(3-(4-chlorophényl)-2-propyn-1-ylthio)-1,2,5-thiadiazol-4-yl)-1-azabicyclo[2.2.2]octane,
l'endo(α)-3-(3-(3-(3,5-difluorophényl)-2-propyn-1-yloxy)-1,2,5-thiadiazol-4-yl)-1-azabicyclo[2.2.1]heptane,
l'endo(α)-3-(3-(3-(3-chloro-5-fluorophényl)-2-propyn-1-yloxy)-1,2,5-thiadiazol-4-yl)-1-azabicyclo[2.2.1] heptane,
l'endo(β)-3-(3-(3-(3-furyl)-2-propyn-1-yloxy)-1,2,5-thiadiazol-4-yl)-1-azabicyclo[2.2.1]heptane,
l'endo(β)-3-(3-(3-(3-cyanophényl)-2-propyn-1-yloxy)-1,2,5-thiadiazol-4-yl)-1-azabicyclo[2.2.1]heptane,
l'endo(β)-3-(3-(3-(3-trifluorométhoxyphényl)-2-propyn-1-yloxy)-1,2,5-thiadiazol-4-yl)-1-azabicyclo[2.2.1] hèptane,
l'endo(β)-3-(3-(3-(3-chlorophényl)-2-propyn-1-yloxy)-1,2,5-thiadiazol-4-yl)-1-azabicyclo[2.2.1]heptane,
l'endo(β)-3-(3-(3-(3,5-dichlorophényl)-2-propyn-1-yloxy)-1,2,5-thiadiazol-4-yl)-1-azabicyclo[2.2.1]heptane,
l'endo(β)-3-(3-(3-(3,5-difluorophényl)-2-propyn-1-yloxy)-1,2,5-thiadiazol-4-yl)-1-azabicyclo[2.2.1]heptane,
l'endo(β)-3-(3-(3-(3-bromophényl)-2-propyn-1-yloxy)-1,2,5-thiadiazol-4-yl)-1-azabicyclo[2.2.1]heptane,
l'endo(β)-3-(3-(3-(3-fluorophényl)-2-propyn-1-yloxy)-1,2,5-thiadiazol-4-yl)-1-azabicyclo[2.2.1]heptane,
l'endo(β)-3-(3-(3-(3-chloro-5-fluorophényl)-2-propyn-1-yloxy)-1,2,5-thiadiazol-4-yl)-1-azabicyclo[2.2.1] heptane,
l'endo(β)-3-(3-(3-(3-bromo-5-fluorophényl)-2-propyn-1-yloxy)-1,2,5-thiadiazol-4-yl)-1-azabicyclo[2.2.1] heptane,
l'endo(β)-3-(3-(3-(5-chloro-2-thiényl)-2-propyn-1-yloxy)-1,2,5-thiadiazol-4-yl)-1-azabicyclo[2.2.1]heptane,
l'endo (α)-3-(3-(3-(4-chlorophényl)-2-propyn-1-ylthio)-1,2,5-thiadiazol-4-yl)-1-azabicyclo[2.2.1]heptane,
l'endo(α)-3-(3-(3-(3,5-difluorophényl)-2-propyn-1-ylthio)-1,2,5-thiadiazol-4-yl)-1-azabicyclo[2.2.1]heptane,
l'endo(α)-3-(3-(3-(3-chloro-5-fluorophényl)-2-propyn-1-ylthio)-1,2,5-thiadiazol-4-yl)-1-azabicyclo[2.2.1] heptane,
le (3S)3-(3-(1-(4-chlorophényl)-4-méthyl-1-pentyn-3-yloxy)-1,2,5-thiadiazol-4-yl)-1-azabicyclo[2.2.2]octane,
le (3S)3-(3-(1-(3-chlorophényl)-4-méthyl-1-pentyn-3-yloxy)-1,2,5-thiadiazol-4-yl)-1-azabicyclo[2-2-2]octane,
le (3S)3-(3-(1-(4-fluorophényl)-4-méthyl-1-pentyn-3-yloxy)-1,2,5-thiadiazol-4-yl)-1-azabicyclo[2.2.2]octane,
le (3S)3-(3-(3-(3,5-dichlorophényl)-2-propyn-1-yloxy)-1,2,5-thiadiazol-4-yl)-1-azabicyclo[2.2.2]octane,
le (3S)3-(3-(3-(3-bromophényl)-2-propyn-1-yloxy)-1,2,5-thiadiazol-4-yl)-1-azabicyclo[2.2.2]octane,
le (3S)3-(3-(3-(3-trifluorométhoxyphényl)-2-propyn-1-yloxy)-1,2,5-thiadiazol-4-yl)-1-azabicyclo[2.2.2]octane,
le (3S)3-(3-(3-(3,5-difluorophényl)-2-propyn-1-yloxy)-1,2,5-thiadiazol-4-yl)-l-azabicyclo[2.2.2]octane,
le (3S)3-(3-(3-(3-chloro-5-fluorophényl)-2-propyn-1-yloxy)-1,2,5-thiadiazol-4-yl)-1-azabicyclo[2.2.2]octane,
le (3S)3-(3-(3-(3-bromo-5-fluorophényl)-2-propyn-1-yloxy)-1,2,5-thiadiazol-4-yl)-1-azabicyclo[2.2.2] octane,
le (3S)3-(3-(3-(5-chloro-2-thiényl)-2-propyn-1-yloxy)-1,2,5-thiadiazol-4-yl)-1-azabicyclo[2.2.2]octane,
l'endo(5R,6S)-6-(3-(3-(2-fluorophényl)-2-propyn-1-ylthio)-1,2,5-thiadiazol-4-yl)-1-azabicyclo[3.2.1]octane,
l'endo(5R,6S)-6-(3-(3-(3-méthylthiophényl)-2-propyn-1-ylthio)-1,2,5-thiadiazol-4-yl)-1-azabicyclo[3.2.1] octane,
l'endo(5R,6S)-6-(3-(1-(3-méthoxyphényl)-4-méthyl-1-pentyn-3-ylthio)-1,2,5-thiadiazol-4-yl)-1-azabicyclo[3.2.1] octane,
l'endo(5R,6S)-6-(3-(l-(3-mêthoxyphényl)-l-pentyn-3-ylthio)-1,2,5-thiadiazol-4-yl)-1-azabicyclo[3.2.1] octane,
l'endo(5R,6S)-6-(3-(1-(2-thiényl)-4-méthyl-1-pentyn-3-ylthio)-1,2,5-thiadiazol-4-yl)-1-azabicyclo[3.2.1] octane,
l'endo(5R,6S)-6-(3-(3-(3,5-difluorophényl)-2-propyn-1-ylthio)-1,2,5-thiadiazol-4-yl)-1-azabicyclo[3.2.1] octane,
l'exo(5R,6R)-6-(3-(1-(3-chlorophényl)-4-méthyl-1-pentyn-3-ylthio)-1,2,5-thiadiazol-4-yl)-1-azabicyclo[3.2.1] octane,
l'exo(5R,6R)-6-(3-(1-(3,5-difluorophényl)-4-méthyl-1-pentyn-3-ylthio)-1,2,5-thiadiazol-4-yl)-1-azabicyclo[3.2.1] octane,
l'exo(5R,6R)-6-(3-(1-(3-cyanophényl)-4-méthyl-1-pentyn-3-ylthio)-1,2,5-thiadiazol-4-yl)-1-azabicyclo[3.2.1] octane,
l'exo(5R,6R)-6-(3-(3-(3-bromophényl)-2-propyn-1-ylthio)-1,2,5-thiadiazol-4-yl)-1-azabicyclo[3.2.1] octane,
l'exo(5R,6R)-6-(3-(3-(3-chloro-5-fluorophényl)-2-propyn-1-ylthio)-1,2,5-thiadiazol-4-yl)-1-azabicyclo[3.2.1] octane,
l'exo(5R,6R)-6-(3-(3-(3-bromo-5-fluorophényl)-2-propyn-1-ylthio)-1,2,5-thiadiazol-4-yl)-1-azabicyclo[3.2.1] octane,
l'exo(5R,6R)-6-(3-(3-(5-pyrimidyl)-2-propyn-1-ylthio)-1,2,5-thiadiazol-4-yl)-1-azabicyclo[3.2.1] octane,
l'exo(5R,6R)-6-(3-(4-méthyl-1-phényl-1-pentyn-3-yloxy)-1,2,5-thiadiazol-4-yl)-1-azabicyclo[3.2.1] octane,
l'exo(5R,6R)-6-(3-(1-(3,5-dichlorophényl)-4-méthyl-1-pentyn-3-yloxy)-1,2,5-thiadiazol-4-yl)-1-azabicyclo [3.2.1]octane,
l'exo(5R,6R)-6-(3-(3-(3-cyanophényl)-2-propyn-1-yloxy)-1,2,5-thiadiazol-4-yl)-1-azabicyclo[3.2.1] octane,
l'exo(5R,6R)-6-(3-(1-(3-chlorophényl)-4-méthyl-1-pentyn-3-yloxy)-1,2,5-thiadiazol-4-yl)-1-azabicyclo[3.2.1] octane,
l'exo(5R,6R)-6-(3-(1-(3-cyanophényl)-4-méthyl-1-pentyn-3-yloxy)-1,2,5-thiadiazol-4-yl)-1-azabicyclo[3.2.1] octane,
l'exo(5R,6R)-6-(3-(1-(3,5-difluorophényl)-4-méthyl-1-pentyn-3-yloxy)-1,2,5-thiadiazol-4-yl)-1-azabicyclo [3.2.1] octane,
l'exo(5R,6R)-6-(3-(3-(3,5-difluorophényl)-2-propyn-1-yloxy)-1,2,5-thiadiazol-4-yl)-1-azabicyclo[3.2.1] octane,
l'exo(5R,6R)-6-(3-(3-(3-bromophényl)-2-propyn-1-yloxy)-1,2,5-thiadiazol-4-yl)-1-azabicyclo[3.2.1] octane,
l'exo(SR,6R)-6-(3-(3-(3-chloro-5-fluorophényl)-2-propyn-1-yloxy)-1,2,5-thiadiazol-4-yl)-1-azabicyclo[3.2.1] octane,
l'exo(5R,6R)-6-(3-(3-(3-bromo-5-fluorophényl)-2-propyn-1-yloxy)-1,2,5-thiadiazol-4-yl)-l-azabicyclo[3.2.1] octane,
l'exo(5R,6R)-6-(3-(1-(2-thiényl)-4-méthyl-1-pentyn-3-yloxy)-1,2,5-thiadiazol-4-yl)-1-azabicyclo[3.2.1]octane,
l'exo(5R,6R)-6-(3-(3-(3-furyl)-2-propyn-1-yloxy)-1,2,5-thiadiazol-4-yl)-1-azabicyclo[3.2.1]octane,
l'endo(β)3-(3-(3-(3,5-difluorophényl)-2-propyn-1-ylthio)-1,2,5-thiadiazol-4-yl)-1-azabicyclo[2.2.1]heptane,
l'endo(β)3-(3-(3-(3-fluorophényl)-2-propyn-1-ylthio)-1,2,5-thiadiazol-4-yl)-1-azabicycio[2.2.1]heptane,
l'endo(β)3-(3-(3-(3-chloro-5-fluorophényl)-2-propyn-1-ylthio)-1,2,5-thiadiazol-4-yl)-1-azabicyclo[2.2.1] heptane,
l'endo(β)3-(3-(3-(3-bromo-5-fluorophényl)-2-propyn-1-ylthio)-1,2,5-thiadiazol-4-yl)-1-azabicycio[2.2.1] heptane,
le (+-)3-(3-(3-(3-chlorophényl)-2-propyn-1-ylthio)-1,2,5-thiadiazol-4-yl)-1-azabicyclo[2.2.2]octane,
le (3S)3-(3-(3-(3-fluorophényl)-2-propyn-1-ylthio)-1,2,5-thiadiazol-4-yl)-1-azabicyclo(2.2.2]octane,
le (3S)3-(3-(3-(3,5-dichlorophényl)-2-propyn-1-ylthio)-1,2,5-thiadiazol-4-yl)-1-azabicyclo[2.2.2]octane,
le (3S)3-(3-(3-(3-bromophényl)-2-propyn-1-ylthio)-1,2,5-thiadiazol-4-yl)-1-azabicyclo[2.2.2]octane,
le (3S)3-(3-(3-(3-trifluorométhoxyphényl)-2-propyn-1-ylthio)-1,2,5-thiadiazol-4-yl)-1-azabicyclo[2.2.2]octane,
le (3S)3-(3-(1-(3,5-difluorophényl)-4-méthyl-1-pentyn-3-ylthio)-1,2,5-thiadiazol-4-yl)-1-azabicyclo[2.2.2] octane,
le (3S)3-(3-(3-(3,5-difluorophényl)-2-propyn-1-ylthio)-1,2,5-thiadiazol-4-yl)-1-azabicyclo[2.2.2]octane,
le (3S)3-(3-(3-(3-bromo-5-fluorophényl)-2-propyn-1-ylthio)-1,2,5-thiadiazol-4-yl)-1-azabicyclo[2.2.2]octane,
le (3S)3-(3-(3-(5-chloro-2-thiényl)-2-propyn-1-ylthio)-1,2,5-thiadiazol-4-yl)-1-azabicyclo[2.2.2]octane,
ou un sel ou produit de solvatation acceptable du point de vue pharmaceutique de celui-ci.

14. Un procédé de préparation d'un composé selon la revendication 1, **caractérisé par** :
a) la réaction de composés aromatiques substitués par de l'iode ou du brome et d'alcools propargyliques en présence d'un catalyseur de pailadium : 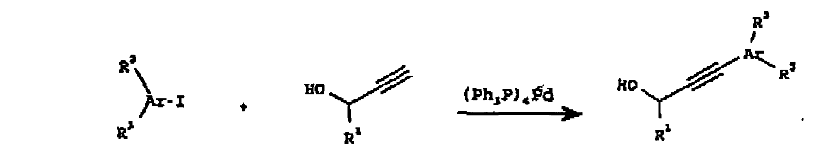 réaction dans laquelle Ar, R¹, R² et R³ ont les significations mentionnées plus haut, lequel alcool peut être amené à réagir avec un 1,2,5-thiadiazole azabicyclique sous des conditions basiques 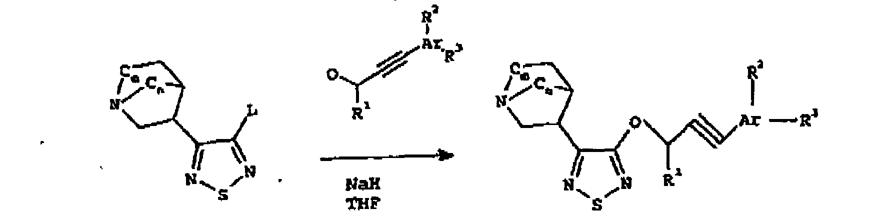 réaction dans laquelle L est un groupe labile, par exemple du chlore ou SO₂R, où R est un radical alkyle ou phényle et Ar, R¹, R², R³, n et m ont les significations mentionnées ci-dessus ; ou
b) les composés dans lesquels X est S peuvent être synthétisés en déplaçant le groupe labile L avec du soufre, par exemple en utilisant NaSH et en alkylant ensuite sur l'atome de soufre avec du P-alcool propargylique, où O-P est un groupe labile, par exemple O-SO₂R ou O-"Mitsunobu", où R a la signification mentionnée plus haut, 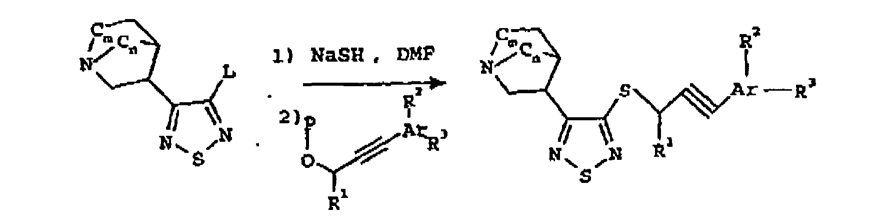 et dans laquelle Ar, R¹, R², R³, n et m ont les significations mentionnées plus haut.

15. Une composition pharmaceutique comprenant un composé selon la revendication 1 conjointement avec un ou plusieurs diluants ou véhicules acceptables du point de vue pharmaceutique.

16. Une composition pharmaceutique destinée à être utilisée dans le traitement d'une maladie du système nerveux central provoquée par un mauvais fonctionnement du système cholinergique muscarinique, comprenant une quantité efficace d'un composé selon la revendication 1 Conjointement avec un diluant ou véhicule acceptable du point de vue pharmaceutique.

17. La composition pharmaceutique selon la revendication 15 ou 16 sous la forme d'une unité de dose orale ou d'une unité de dose parentérale.

18. La composition pharmaceutique selon la revendication 17, dans laquelle ladite unité de dose renferme d'environ 0,1 à environ 100 mg de composé selon la revendication 1.

19. Utilisation d'un composé selon la revendication 1 ou d'un sel acceptable du point de vue pharmaceutique de celui-ci pour la préparation d'un médicament pour le traitement d'une maladie du système nerveux central provoquée par un mauvais fonctionnement du système cholinergique muscarinique.

20. Utilisation d'un composé selon la revendication 1 ou d'un sel acceptable du point de vue pharmaceutique de celui-ci pour la préparation d'un médicament pour le traitement d'une condition associée à la modulation d'un récepteur cholinergique muscarinique.

21. Utilisation d'un composé selon la revendication 1 ou d'un sel acceptée du point de vne pharmaceutique de celui-ci pour la préparation d'un médicament pour l'interaction avec un récepteur cholinergique muscarinique.
